# EUROPEAN PATENT APPLICATION

(11) **EP 4 725 972 A1**
(43) Date of publication of application: **15.04.2026**
(21) Application number: 24819430.0
(22) Date of filing: 07.06.2024
(51) Int. Cl.: C08G 18/08, C07C 263/16, C07C 263/18, C07C 265/14

(54) **POLYISOCYANATE COMPOSITION AND METHOD FOR PRODUCING ISOCYANATE COMPOUND**

(30) Priority: 08.06.2023 JP 2023094699; 08.06.2023 JP 2023094710; 08.06.2023 JP 2023094723; 08.06.2023 JP 2023094942; 08.06.2023 JP 2023094958
(71) Applicant: ASAHI KASEI KABUSHIKI KAISHA, Tokyo 100-0006 (JP)
(72) Inventor: INADA, Hiroshi, Tokyo 100-0006 (JP); KOSUGI, Yuji, Tokyo 100-0006 (JP); FUJIWARA, Atsushi, Tokyo 100-0006 (JP); ONISHI, Kazuhiro, Tokyo 100-0006 (JP)
(74) Representative: D Young & Co LLP
(86) International application number: PCT/JP2024/020945
(87) International publication number: WO 2024/253203

(57) **Abstract**

Disclosed is a polyisocyanate composition containing at least one or more compounds represented by any one of general formulas (I), (II), and (III), and a polyisocyanate compound.

Also disclosed is a method for producing an isocyanate compound, the method including a reaction step of decomposing a blocked isocyanate compound into a blocking agent and an isocyanate compound by heat treatment in the presence of a compound having a structure represented by either one or both of general formulas (I) and (II), thereby obtaining the isocyanate compound.

## Description

### TECHNICAL FIELD

The present invention relates to a polyisocyanate composition and a method for producing an isocyanate compound.

Priority is claimed on Japanese Patent Application Nos. 2023-094942, 2023-094699, 2023-094710, 2023-094723, and 2023-094958, filed June 8, 2023, the contents of which are incorporated herein by reference.

### BACKGROUND ART

Polyisocyanate compounds are used in polyurethane synthesis and as curing agents and the like, and are utilized in a wide range of fields, including flexible foams, rigid foams, elastomers, adhesives, coating materials, and binders.

For example, Patent Document 1 describes an isocyanate composition containing a trifunctional or higher isocyanate compound and a compound having at least one unsaturated bond other than an unsaturated bond constituting an aromatic ring.

Patent Document 2 describes a polyisocyanate composition containing a polyisocyanate and a compound having an unsaturated bond, or at least one inert compound selected from the group consisting of a hydrocarbon compound, an ether compound, a sulfide compound, a halogenated hydrocarbon compound, a silicon-containing hydrocarbon compound, a silicon-containing ether compound, and a silicon-containing sulfide compound.

Further, isocyanates are widely used as raw materials for the production of polyurethane foams, coating materials, adhesives, and the like. The main industrial method for producing isocyanates involves a reaction of an amine compound with phosgene (phosgene process), and almost all the amount produced worldwide is produced by the phosgene process. However, the phosgene process has a number of problems concerning phosgene as the raw material and hydrogen chloride as the by-product.

From such a background, a method for producing isocyanates without using phosgene is desired. For example, Patent Document 3 describes a method for producing 1,6-hexamethylene diisocyanate by thermally decomposing 1,6-hexamethylene dicarbamate in the presence of a specific catalyst. In addition, Patent Document 4 describes a method for producing a diisocyanate compound by reacting a diamine with dimethyl carbonate in the presence of an alkali catalyst to synthesize a urethane compound, and then thermally decomposing the urethane compound in the presence of a catalyst.

Further, isocyanates are highly reactive and react easily with compounds such as water. For this reason, for the sake of improving stability, there are cases where isocyanates are converted into blocked isocyanates, and in use, are regenerated and used by dissociating a blocking agent by heating. Blocked isocyanates exhibit low reactivity with active hydrogen compounds, can be stored stably, and are less toxic than isocyanates, making them useful as one-component type coating materials, adhesives, and compounds for molding.

In addition, blocked isocyanates have a property of dissociating into an isocyanate and a blocking agent due to thermal decomposition. For this reason, a blocked isocyanate can be decomposed into an isocyanate and a blocking agent by thermal decomposition, and the obtained isocyanate and blocking agent can be separated after or simultaneously with thermal decomposition. The separated isocyanate and blocking agent are useful because they can also be used as raw materials in the case of producing isocyanates.

Various methods for producing blocked isocyanates are known. Examples thereof include: a method for producing a blocked isocyanate by directly reacting an isocyanate with a blocking agent; a method for producing a blocked isocyanate by reacting, with a blocking agent, a carbamic acid chloride obtained by reacting an amine with phosgene; a method for producing a blocked isocyanate by reacting carbamic acid, a blocking agent, and a condensing agent; a method for producing a blocked isocyanate containing a compound derived from a carbonic acid derivative by reacting an amine with the carbonic acid derivative; and a method for producing a blocked isocyanate by reacting an amine with a carbonic acid derivative and a blocking agent.

In particular, when producing a blocked isocyanate using an amine as a raw material, a side reaction may occur that generates the resulting blocked isocyanate and a ureylene group, as described in Patent Document 5.

When producing an isocyanate by thermally decomposing a blocked isocyanate composition containing such a ureylene group into an isocyanate and a blocking agent and then separating the obtained isocyanate and blocking agent after or simultaneously with the thermal decomposition, the resulting isocyanate may react with the ureylene group to form biuret, which may result in an increase in the amount of by-products produced.

### Citation List

### Patent Documents

Patent Document 1: International Patent Publication No. 2018/070539
Patent Document 2: International Patent Publication No. 2014/069605
Patent Document 3: Japanese Unexamined Patent Application, First Publication No. Hei 6-239826
Patent Document 4: Japanese Unexamined Patent Application, First Publication No. Sho 64-85956
Patent Document 5: Japanese Unexamined Patent Application, First Publication No. 2022-180170

### SUMMARY OF INVENTION

### Technical Problem

When a polyisocyanate compound is utilized in a field where appearance quality is required, for example, as a raw material for polyurethane and the like, it is important that the polyisocyanate compound exhibits minimal coloration. However, in general, there is a tendency that isocyanates are oxidized by oxygen and the like in the air, resulting in deterioration or coloration. Further, also when producing an isocyanate polymer by polymerization of a diisocyanate, coloration of the isocyanate tends to occur due to the catalyst or solvent used in the polymerization reaction.

Moreover, in a method for producing an isocyanate compound, when a blocked isocyanate compound is thermally decomposed in the presence of a catalyst to obtain an isocyanate compound, improving the thermal decomposition rate involves an increase in the amount of by-products produced in some cases.

One aspect of the present invention has been made in view of the above circumstances, and has an object to provide a polyisocyanate composition that exhibits excellent storage stability and coloration inhibition.

One aspect of the present invention has been made in view of the above circumstances, and has an object to provide a method for producing an isocyanate compound that can improve the thermal decomposition rate of a blocked isocyanate compound without increasing the amount of by-products produced.

### Solution to Problem

That is, the present invention includes the following aspects.
[1] A polyisocyanate composition containing at least one or more compounds represented by any one of the following general formulas (I), (II), and (III), and a polyisocyanate compound, 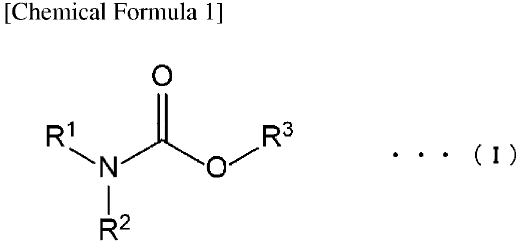 (in the general formula (I), R¹ and R² each independently represent a monovalent organic group; R¹ and R² may each independently form a ring structure via a carbon-carbon bond, a carbon-oxygen-carbon bond, or a carbon-nitrogen-carbon bond; and R³ represents an aliphatic hydrocarbon group or a hydrocarbon group having an aromatic group), 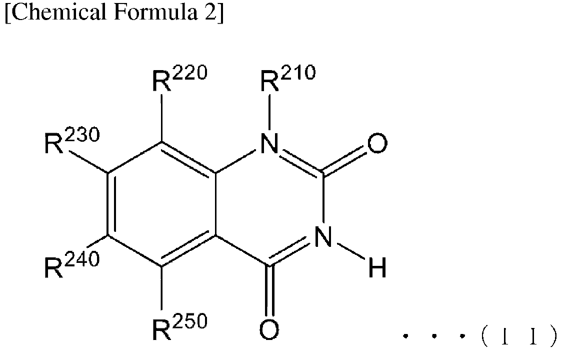 (in the general formula (II), R²¹⁰ represents a monovalent organic group, and R²²⁰, R²³⁰, R²⁴⁰, and R²⁵⁰ each independently represent a monovalent organic group or hydrogen; and R²¹⁰ and R²²⁰ may be bonded to each other to form a ring structure via a carbon-carbon bond, a carbon-oxygen-carbon bond, or a carbon-nitrogen-carbon bond), 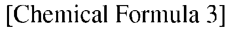 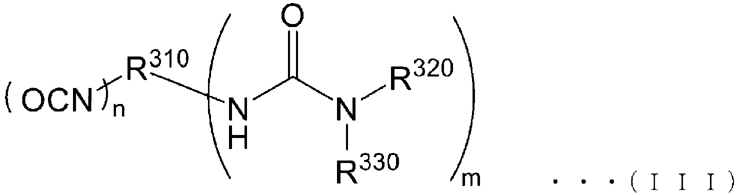 (in the general formula (III), R³¹⁰ is an (n + m )-valent organic group, n is an integer from 0 to 12, m is an integer from 1 to 12, n + m is an integer of 13 or less, and R³²⁰ and R³³⁰ each independently represent a monovalent organic group; R³²⁰ and R³³⁰ may be bonded to each other to form a ring structure via a carbon-carbon bond, a carbon-oxygen-carbon bond, or a carbon-nitrogen-carbon bond; and at least one of R³²⁰ and R³³⁰ has an aromatic group.)
[2] The polyisocyanate composition according to [1], containing 1.0 ppm by mass or more and 1.0 × 10⁴ ppm by mass or less of at least one or more compounds represented by any one of the aforementioned general formulas (I), (II), and (III), based on a total mass of the aforementioned polyisocyanate compound.
[3] A method for producing an isocyanate compound, the method including a reaction step of decomposing a blocked isocyanate compound into a blocking agent and an isocyanate compound by heat treatment in the presence of a compound having a structure represented by either one or both of the following general formulas (I) and (II), thereby obtaining the isocyanate compound: 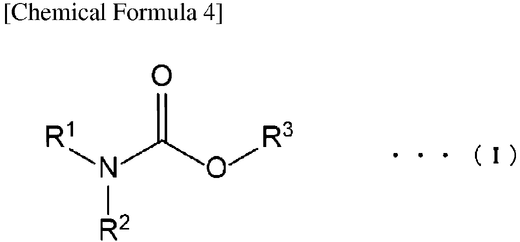 (in the general formula (I), R¹ and R² each independently represent a monovalent organic group; R¹ and R² may each independently form a ring structure via a carbon-carbon bond, a carbon-oxygen-carbon bond, or a carbon-nitrogen-carbon bond; and R³ represents an aliphatic hydrocarbon group or a hydrocarbon group having an aromatic group), 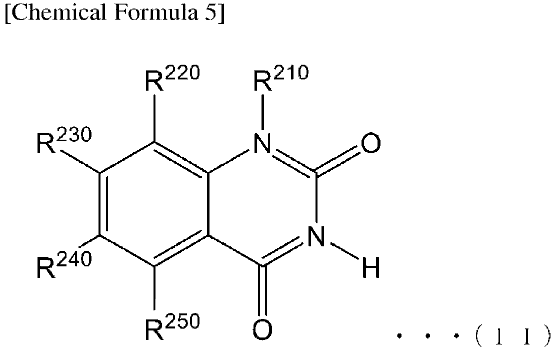 (in the general formula (II), R²¹⁰ represents a monovalent organic group, and R²²⁰, R²³⁰, R²⁴⁰, and R²⁵⁰ each independently represent a monovalent organic group or hydrogen; and R²¹⁰ and R²²⁰ may be bonded to each other to form a ring structure via a carbon-carbon bond, a carbon-oxygen-carbon bond, or a carbon-nitrogen-carbon bond.)
[4] The method for producing an isocyanate compound according to [3], wherein the aforementioned blocking agent contains one or more compounds selected from the group consisting of a hydroxy compound, an amine compound, and ammonia.
[5] The method for producing an isocyanate compound according to [3] or [4], wherein the aforementioned isocyanate compound is an isocyanate compound represented by the following general formula (IV): 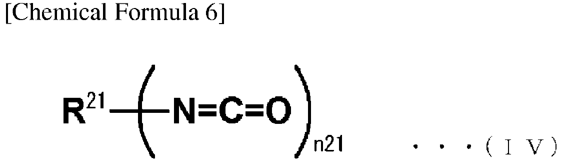 (in the general formula (IV), R²¹ is an n21-valent organic group; and n21 is an integer from 1 to 12.)
[6] The method for producing an isocyanate compound according to [3] or [4], wherein the aforementioned blocking agent is an aromatic hydroxy compound represented by the following general formula (V): 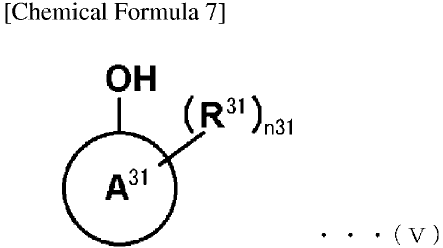 (in the general formula (V), a ring A³¹ represents an aromatic hydrocarbon ring having 6 to 20 carbon atoms; R³¹ represents a hydrogen atom, a halogen atom, a carboxy group, an alkyl group having 1 to 20 carbon atoms, an alkoxy group having 1 to 20 carbon atoms, an alkyloxycarbonyl group having 1 to 20 carbon atoms, an alkylcarbonyloxy group having 1 to 20 carbon atoms, an aryl group having 6 to 20 carbon atoms, an aryloxy group having 6 to 20 carbon atoms, an aralkyl group having 7 to 20 carbon atoms, or an aralkyloxy group having 7 to 20 carbon atoms; R³¹ may be bonded with the ring A³¹ to form a ring structure; and n31 is an integer from 1 to 10.)
[7] The method for producing an isocyanate compound according to [3] or [4], wherein the aforementioned blocking agent is an aliphatic hydroxy compound represented by the following general formula (VI): 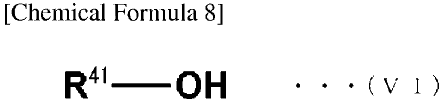 (in the general formula (VI), R⁴¹ represents a substituted or unsubstituted aliphatic hydrocarbon group having 1 to 24 carbon atoms, which may have an ether group, a carbonyl group, or an ester group.)
[8] The method for producing an isocyanate compound according to [3] or [4], wherein the aforementioned blocking agent is a secondary amine compound represented by the following general formula (VII): 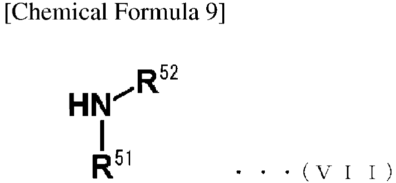 (in the general formula (VII), R⁵¹ and R⁵² each independently represent a monovalent organic group; R⁵¹ and R⁵² may be bonded to each other to form a ring structure via a carbon-carbon bond, a carbon-oxygen-carbon bond, or a carbon-nitrogen-carbon bond.)
[9] The method for producing an isocyanate compound according to [3] or [4], wherein in the aforementioned reaction step, an amount of a compound having a structure represented by either one or both of the aforementioned general formulas (I) and (II) present is 1 ppm by mass or more with respect to the aforementioned blocked isocyanate compound.

### Advantageous Effects of Invention

According to the polyisocyanate composition of the above aspect, it is possible to provide a polyisocyanate composition with excellent storage stability and coloration inhibition.

According to the method for producing an isocyanate compound of the above aspect, it is possible to provide a method for producing an isocyanate compound that can improve the thermal decomposition rate of a blocked isocyanate compound without increasing the amount of by-products produced.

### DESCRIPTION OF EMBODIMENTS

Hereinafter, an embodiment for carrying out the present invention (hereinafter, referred to as the "present embodiment") will be described in detail. The present embodiment below is an illustration for explaining the present invention, and the present invention is not limited to the present embodiment below. The present invention can be carried out with appropriate modifications within the scope of the gist thereof.

It should be noted that in the present specification, the term "active hydrogen" refers to a hydrogen atom bonded to an oxygen atom, sulfur atom, or nitrogen atom, and a hydrogen atom of an active methylene group. Examples thereof include hydrogen atoms contained in atomic groups such as -OH groups, -C(=O)OH groups, -SH groups, - NH₂ groups, -NH- groups, and -C(=O)-C(-H)₂-C(=O)- groups.

### <Polyisocyanate composition>

A polyisocyanate composition of the present embodiment contains at least one or more compounds represented by any one of general formulas (I), (II), and (III), and a polyisocyanate compound.

The polyisocyanate composition of the present embodiment can contain an optional component other than compounds (I), (II), and (III) and the polyisocyanate compound, depending on the application, purpose, and the like.

The polyisocyanate composition of the present embodiment can be used as a material for synthesizing polyurethane, a curing agent, and the like.

The polyisocyanate composition of the present embodiment can be utilized in a wide range of fields, including flexible foams, rigid foams, elastomers, adhesives, coating materials, and binders.

A polyisocyanate composition 1 of the present embodiment contains a compound represented by the general formula (I) and a polyisocyanate compound. In the following description, a "compound represented by the general formula (I)" may be referred to as a "compound (I)."

A polyisocyanate composition 2 of the present embodiment contains a compound represented by the general formula (II) and a polyisocyanate compound. In the following description, a "compound represented by the general formula (II)" may be referred to as a "compound (II)."

A polyisocyanate composition 3 of the present embodiment contains a compound represented by the general formula (III) and a polyisocyanate compound. In the following description, a "compound represented by the general formula (III)" may be referred to as a "compound (III)."

A polyisocyanate composition 4 of the present embodiment contains two or more compounds among the compounds represented by the general formulas (I), (II), and (III), and a polyisocyanate compound.

One aspect of the polyisocyanate composition 4 contains compounds represented by the general formulas (I), (II), and (III) and a polyisocyanate compound.

### <<Polyisocyanate composition 1>>

The polyisocyanate composition 1 of the present embodiment contains the compound (I) and a polyisocyanate compound.

### [Compound (I)]

The compound (I) is represented by the following general formula (I). 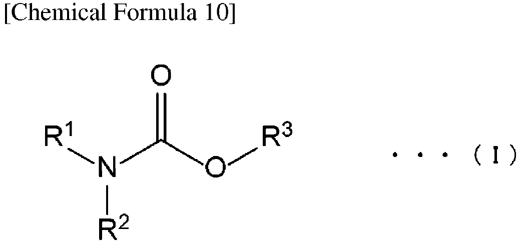 (in the general formula (I), R¹ and R² each independently represent a monovalent organic group; R¹ and R² may each independently form a ring structure via a carbon-carbon bond, a carbon-oxygen-carbon bond, or a carbon-nitrogen-carbon bond; and R³ represents an aliphatic hydrocarbon group or a hydrocarbon group having an aromatic group.)

### [R¹ and R²]

In the above general formula (I), R¹ and R² each independently represent a monovalent organic group. R¹ and R² may each independently form a ring structure via a carbon-carbon bond, a carbon-oxygen-carbon bond, or a carbon-nitrogen-carbon bond.

In particular, R¹ and R² each independently preferably represent a monovalent aliphatic hydrocarbon group having 1 to 70 carbon atoms, which may have a substituent, or a monovalent aromatic hydrocarbon group having 6 to 70 carbon atoms, which may have a substituent.

When an aliphatic hydrocarbon group is selected for at least one of R¹ and R² the number of carbon atoms is preferably from 1 to 70, more preferably from 1 to 20, still more preferably from 1 to 12, and particularly preferably from 1 to 10.

Examples of the aliphatic hydrocarbon group represented by R¹ and R² include a linear alkyl group, branched alkyl group, and cycloalkyl group, which may be unsubstituted or substituted. Examples of the substituent on these aliphatic hydrocarbon groups include a hydroxyl group, a cyano group, and a halogen atom. Examples of the halogen atom as a substituent include a fluorine atom, a chlorine atom, a bromine atom, and an iodine atom.

Specific examples of the aliphatic hydrocarbon group represented by R¹ and R² include a methyl group, an ethyl group, an n-propyl group, an isopropyl group, an n-butyl group, an isobutyl group, a sec-butyl group, a tert-butyl group, each isomer of a pentyl group, each isomer of a hexyl group, each isomer of a heptyl group, each isomer of an octyl group, each isomer of a nonyl group, each isomer of a decyl group, a cyclopentyl group, and a cyclohexyl group. Among these, an unsubstituted aliphatic hydrocarbon group having 1 to 12 carbon atoms is preferred, and a methyl group, an ethyl group, an n-propyl group, an isopropyl group, an n-butyl group, or an isobutyl group is preferred.

When an aromatic hydrocarbon group is selected for at least one of R¹ and R², the number of carbon atoms is preferably from 6 to 70, more preferably from 6 to 20, still more preferably from 6 to 12, and particularly preferably from 6 to 10.

Examples of the aromatic hydrocarbon group represented by R¹ and R² include an aryl group and aralkyl group, which may be unsubstituted or substituted. Examples of the substituent on these aromatic hydrocarbon groups include an aliphatic hydrocarbon group, a hydroxyl group, a cyano group, and a halogen atom. Examples of the aliphatic hydrocarbon group selected as the substituent on the aromatic hydrocarbon group represented by R¹ and R² include the same groups as those exemplified as the aliphatic hydrocarbon group represented by R¹ and R². Examples of the halogen atom as a substituent include a fluorine atom, a chlorine atom, a bromine atom, and an iodine atom.

Specific examples of the aromatic hydrocarbon group represented by R¹ and R² include a phenyl group, a naphthyl group, an anthryl group, a pyrenyl group, a phenanthryl group, an o-tolyl group, an m-tolyl group, a p-tolyl group, a benzyl group, and a phenethyl group.

When R¹ and R² are bonded to each other to form a ring structure, a group formed by R¹ and R² bonding to each other is a divalent organic group. The group formed by R¹ and R² bonding to each other is preferably a substituted or unsubstituted divalent aliphatic hydrocarbon group having 1 to 70 carbon atoms or divalent aromatic hydrocarbon group having 6 to 70 carbon atoms, which may have an ether group, a carbonyl group, an ester group, an imino group (-NH-), an amide group, or an imide group.

### [R³]

In the general formula (I), R³ represents an aliphatic hydrocarbon group or a hydrocarbon group having an aromatic group.

Specific examples of the aliphatic hydrocarbon group represented by R³ include a methyl group, an ethyl group, an n-propyl group, an isopropyl group, an n-butyl group, an isobutyl group, a sec-butyl group, a tert-butyl group, each isomer of a pentyl group, each isomer of a hexyl group, each isomer of a heptyl group, each isomer of an octyl group, each isomer of a nonyl group, each isomer of a decyl group, a cyclopentyl group, and a cyclohexyl group. Among these, an unsubstituted aliphatic hydrocarbon group having 1 to 12 carbon atoms is preferred, and a methyl group, an ethyl group, an n-propyl group, an isopropyl group, an n-butyl group, or an isobutyl group is preferred.

When an aromatic hydrocarbon group is selected for R³, the number of carbon atoms is preferably from 6 to 70, more preferably from 6 to 20, still more preferably from 6 to 12, and particularly preferably from 6 to 10.

Examples of the aromatic hydrocarbon group represented by R³ include an aryl group and aralkyl group, which may be unsubstituted or substituted. Examples of the substituent on these aromatic hydrocarbon groups include an aliphatic hydrocarbon group, a hydroxyl group, a cyano group, and a halogen atom. Examples of the aliphatic hydrocarbon group selected as the substituent on the aromatic hydrocarbon group represented by R³ include the same groups as those exemplified as the aliphatic hydrocarbon group represented by R³. Examples of the halogen atom as a substituent include a fluorine atom, a chlorine atom, a bromine atom, and an iodine atom.

Specific examples of the aromatic hydrocarbon group represented by R³ include a phenyl group, a naphthyl group, an anthryl group, a pyrenyl group, a phenanthryl group, an o-tolyl group, an m-tolyl group, a p-tolyl group, a benzyl group, and a phenethyl group.

The compound (I) is preferably a compound represented by the following general formula (I-1). 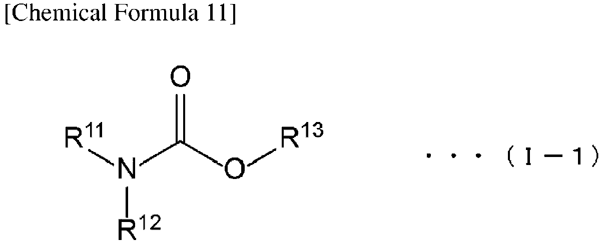 [In the general formula (I-1), R¹¹ is a substituted or unsubstituted monovalent aliphatic hydrocarbon group having 1 to 70 carbon atoms, or a substituted or unsubstituted monovalent aromatic hydrocarbon group having 6 to 70 carbon atoms; R¹² is a substituted or unsubstituted monovalent aliphatic hydrocarbon group having 1 to 20 carbon atoms, or a substituted or unsubstituted monovalent aromatic hydrocarbon group having 6 to 70 carbon atoms; and R¹³ is a substituted or unsubstituted monovalent aliphatic hydrocarbon group having 1 to 20 carbon atoms, or a substituted or unsubstituted monovalent aromatic hydrocarbon group having 6 to 70 carbon atoms.]

### [R¹¹]

In the general formula (I-1), R¹¹ is a substituted or unsubstituted monovalent aliphatic hydrocarbon group having 1 to 70 carbon atoms, or a substituted or unsubstituted monovalent aromatic hydrocarbon group having 6 to 70 carbon atoms. Specific examples of R¹¹ include a methyl group, an ethyl group, an n-propyl group, an isopropyl group, an n-butyl group, an isobutyl group, a sec-butyl group, a tert-butyl group, each isomer of a pentyl group, each isomer of a hexyl group, each isomer of a heptyl group, each isomer of an octyl group, each isomer of a nonyl group, each isomer of a decyl group, a cyclopentyl group, a cyclohexyl group, a phenyl group, a benzyl group, an o-tolyl group, an m-tolyl group, and a p-tolyl group. Among these, an unsubstituted aliphatic hydrocarbon group having 1 to 12 carbon atoms is preferred, and a methyl group, an ethyl group, an n-propyl group, an isopropyl group, an n-butyl group, or an isobutyl group is preferred.

### [R¹²]

In the general formula (I-1), R¹² is a substituted or unsubstituted monovalent aliphatic hydrocarbon group having 1 to 20 carbon atoms, or a substituted or unsubstituted monovalent aromatic hydrocarbon group having 6 to 70 carbon atoms. Specific examples of R¹² include a hydrogen atom, a methyl group, an ethyl group, an n-propyl group, an isopropyl group, an n-butyl group, an isobutyl group, a sec-butyl group, a tert-butyl group, each isomer of a pentyl group, each isomer of a hexyl group, each isomer of a heptyl group, each isomer of an octyl group, each isomer of a nonyl group, each isomer of a decyl group, a cyclopentyl group, a cyclohexyl group, a phenyl group, a benzyl group, an o-tolyl group, an m-tolyl group, and a p-tolyl group. Among these, an unsubstituted aliphatic hydrocarbon group having 1 to 12 carbon atoms is preferred, and a methyl group, an ethyl group, an n-propyl group, an isopropyl group, an n-butyl group, or an isobutyl group is preferred.

### [R¹³]

In the general formula (I-1), R¹³ is a substituted or unsubstituted monovalent aliphatic hydrocarbon group having 1 to 20 carbon atoms, or a substituted or unsubstituted monovalent aromatic hydrocarbon group having 6 to 70 carbon atoms. Examples of R¹³ include a monovalent aliphatic hydrocarbon group having 1 to 12 carbon atoms and a monovalent aromatic hydrocarbon group having 6 to 12 carbon atoms, which may be substituted or unsubstituted. Specific examples of R¹³ include a methyl group, an ethyl group, an n-propyl group, an isopropyl group, an n-butyl group, an isobutyl group, a sec-butyl group, a tert-butyl group, each isomer of a pentyl group, each isomer of a hexyl group, each isomer of a heptyl group, each isomer of an octyl group, each isomer of a nonyl group, each isomer of a decyl group, a cyclopentyl group, a cyclohexyl group, a phenyl group, a benzyl group, an o-tolyl group, an m-tolyl group, and a p-tolyl group. Among these, an unsubstituted aliphatic hydrocarbon group having 1 to 12 carbon atoms is preferred, and a methyl group, an ethyl group, an n-propyl group, an isopropyl group, an n-butyl group, or an isobutyl group is preferred.

In the general formula (I-1), when R¹³ is a monovalent aromatic hydrocarbon group having 6 to 12 carbon atoms, it is preferably a phenyl group which may have a substituent. Examples of the substituent that the phenyl group represented by R¹³ may have include an aliphatic hydrocarbon group or an aromatic hydrocarbon group, such as an alkyl group having 1 to 12 carbon atoms, an aryl group having 6 to 12 carbon atoms, and an aralkyl group having 7 to 12 carbon atoms.

When the substituent on a benzene ring has an ether group, examples thereof include an alkoxy group having 1 to 12 carbon atoms, an aryloxy group having 6 to 12 carbon atoms, or an aralkyloxy group having 7 to 12 carbon atoms. When the substituent on a benzene ring has a carbonyl group, examples thereof include an alkylcarbonyl group having 1 to 12 carbon atoms, an arylcarbonyl group having 6 to 12 carbon atoms, or an aralkylcarbonyl group having 7 to 12 carbon atoms.

When the substituent on a benzene ring has an ester group, examples thereof include an alkoxycarbonyl group or alkylcarbonyloxy group having 1 to 12 carbon atoms, an aryloxycarbonyl group or arylcarbonyloxy group having 6 to 12 carbon atoms, or an aralkyloxycarbonyl group or aralkylcarbonyloxy group having 7 to 12 carbon atoms.

Specific examples of the substituent on a benzene ring include a methyl group, an ethyl group, an n-propyl group, an isopropyl group, an n-butyl group, an isobutyl group, a sec-butyl group, a tert-butyl group, each isomer of a pentyl group, each isomer of a hexyl group, each isomer of a heptyl group, each isomer of an octyl group, each isomer of a nonyl group, each isomer of a decyl group, a cyclopentyl group, a cyclohexyl group, a phenyl group, a benzyl group, an o-tolyl group, an m-tolyl group, a p-tolyl group, and a cumyl group. Among these, an unsubstituted aliphatic hydrocarbon group having 1 to 12 carbon atoms is preferred, and a methyl group, an ethyl group, an n-propyl group, an isopropyl group, an n-butyl group, or an isobutyl group is preferred.

The compound (I) is preferably a compound represented by the following general formula (I-2). 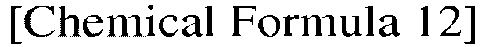 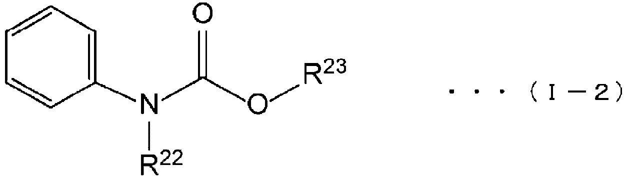 [In the general formula (I-2), R²² is a substituted or unsubstituted monovalent aliphatic hydrocarbon group having 1 to 20 carbon atoms, or a substituted or unsubstituted monovalent aromatic hydrocarbon group having 6 to 70 carbon atoms; and R²³ is a substituted or unsubstituted monovalent aliphatic hydrocarbon group having 1 to 20 carbon atoms, or a substituted or unsubstituted monovalent aromatic hydrocarbon group having 6 to 70 carbon atoms.]

### [R²²]

In the general formula (I-2), R²² is a substituted or unsubstituted monovalent aliphatic hydrocarbon group having 1 to 20 carbon atoms, or a substituted or unsubstituted monovalent aromatic hydrocarbon group having 6 to 70 carbon atoms, and specific descriptions thereof are the same as those for R¹² above.

In the general formula (I-2), R²² is preferably a linear or branched alkyl group having 1 to 10 carbon atoms, or an unsubstituted phenyl group.

### [R²³]

In the general formula (I-2), R²³ is a substituted or unsubstituted monovalent aliphatic hydrocarbon group having 1 to 20 carbon atoms, or a substituted or unsubstituted monovalent aromatic hydrocarbon group having 6 to 70 carbon atoms, and specific descriptions thereof are the same as those for R¹³ above.

In the general formula (I-2), R²³ is preferably a linear or branched alkyl group having 1 to 10 carbon atoms, or a substituted or unsubstituted phenyl group. When R²³ is a phenyl group having a substituent, examples of the substituent include a linear or branched alkyl group having 1 to 10 carbon atoms.

The compound (I) acts as an ultraviolet absorber. For this reason, according to a polyisocyanate composition containing the compound (I), it is possible to reduce the discoloration and denaturation of a polyisocyanate compound due to ultraviolet rays, even when stored outdoors.

Since the compound (I) inhibits the excitation of a chromophore by light, it is less likely to generate radicals when irradiated with light. Since the compound (I) acts as a stabilizer during storage, a polyisocyanate composition containing the compound (I) exhibits excellent storage stability.

Although it is preferable to increase the content of the compound (I) in order to improve the storage stability and coloration inhibition of the polyisocyanate composition, when the content of the compound (I) is too high, the production cost, applications, and the like of the polyisocyanate composition may be adversely affected.

The polyisocyanate composition 1 preferably contains 1.0 ppm by mass or more and 5.0 × 10⁴ ppm by mass or less of compound (I), and more preferably 1.0 ppm by mass or more and 1.0 × 10⁴ ppm by mass or less of compound (I), based on the total mass of the polyisocyanate compounds.

As the compound (I), a commercially available product may be used, or a compound synthesized by a known production method may be used.

Examples of the commercially available product of the compound (I) include N-methylcarbanilic acid phenyl ester 95% (product name, product number: AG00AJDF; CAS number: 13599-69-4; manufactured by Angene International Ltd.)

As a method for synthesizing the compound (I), the corresponding chloroformic acid ester and the corresponding amine are stirred from room temperature to reflux conditions in an appropriate solvent in the presence of an agent such as triethylamine for neutralizing hydrochloric acid generated as a by-product. Alternatively, the corresponding carbamoyl chloride and the corresponding alcohol are stirred from room temperature to reflux conditions in an appropriate solvent in the presence of an agent such as triethylamine for neutralizing hydrochloric acid generated as a by-product. If necessary, a catalyst such as N,N-dimethyl-4-aminopyridine (DMAP) may be used. The produced compound (I) is isolated and purified using known methods.

### <<Polyisocyanate composition 2>>

The polyisocyanate composition 2 of the present embodiment contains the compound (II) and a polyisocyanate compound.

### [Compound (II)]

The compound (II) is represented by the following general formula (II). The compound (II) may also be referred to as a "quinazolinedione compound (II)." 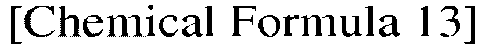 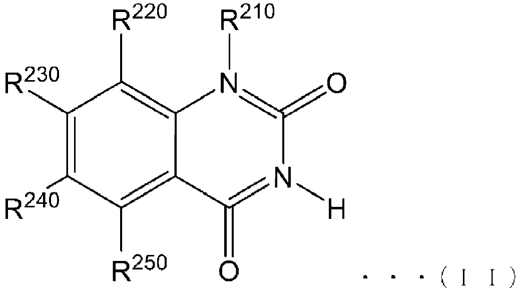 (In the general formula (II), R²¹⁰ is a monovalent organic group, and R²²⁰, R²³⁰, R²⁴⁰, and R²⁵⁰ each independently represent a monovalent organic group or hydrogen; and R²¹⁰ and R²²⁰ may be bonded to each other to form a ring structure via a carbon-carbon bond, a carbon-oxygen-carbon bond, or a carbon-nitrogen-carbon bond.)

The quinazolinedione compound (II) has a quinazoline-2,4(1H,3H)-dione structure (hereinafter sometimes referred to as a "quinazolinedione structure"). The quinazolinedione structure contains an active hydrogen atom in a -C(=O)-NH-C(=O)-group.

However, the -C(=O)-NH-C(=O)- group contained in the quinazolinedione structure may exhibit tautomerism represented by -C(-OH)=N-C(=O)- or -C(=O)-N=C(-OH)-.

The inventors of the present invention and others discovered that the problems of thermal denaturation and coloration can be solved by producing a blocked isocyanate in the presence of a specific amine compound having an aromatic group, thereby leading to completion of the present invention.

Conventionally, compounds having an amino group bonded to a carbon atom having aromaticity have been known as compounds that are easily oxidized and cause coloration. Therefore, although compounds having an amino group bonded to a carbon atom having aromaticity have been used at times in products where coloration is not an issue, such as rubber degradation inhibitors, they have not been used in products where coloration is an issue.

On the other hand, according to the polyisocyanate composition 2 of the present embodiment, it has now been surprisingly discovered for the first time that even when using a specific amine compound having an aromatic group, far from causing the problem of coloration, an exceptionally remarkable effect of improving (inhibiting) thermal denaturation and coloration is exhibited.

### [R²¹⁰, R²²⁰, R²³⁰, R²⁴⁰, and R²⁵⁰]

In the above general formula (II), R²¹⁰ is a monovalent organic group, and R²²⁰, R²³⁰, R²⁴⁰, and R²⁵⁰ each independently represent a monovalent organic group or hydrogen. R²¹⁰ and R²²⁰ may be bonded to each other to form a ring structure via a carbon-carbon bond, a carbon-oxygen-carbon bond, or a carbon-nitrogen-carbon bond.

In particular, R²¹⁰ is preferably a substituted or unsubstituted monovalent aliphatic hydrocarbon group having 1 to 70 carbon atoms or monovalent aromatic hydrocarbon group having 6 to 70 carbon atoms, which may have an ether group, a carbonyl group, an ester group, an imino group (-NH-), an amide group, or an imide group.

Further, R²²⁰, R²³⁰, R²⁴⁰, and R²⁵⁰ each preferably independently represents a substituted or unsubstituted monovalent aliphatic hydrocarbon group having 1 to 70 carbon atoms or monovalent aromatic hydrocarbon group having 6 to 70 carbon atoms, which may have an ether group, a carbonyl group, an ester group, an imino group (-NH-), an amide group, or an imide group; or hydrogen.

When an aliphatic hydrocarbon group is selected for at least one of R²¹⁰, R²²⁰, R²³⁰, R²⁴⁰, and R²⁵⁰, the number of carbon atoms is preferably from 1 to 70, more preferably from 1 to 20, still more preferably from 1 to 12, and particularly preferably from 1 to 10.

Examples of the aliphatic hydrocarbon group represented by R²¹⁰, R²²⁰, R²³⁰, R²⁴⁰, and R²⁵⁰ include a linear alkyl group, branched alkyl group, and cycloalkyl group, which may be unsubstituted or substituted. Examples of the substituent on these aliphatic hydrocarbon groups include a hydroxyl group, a cyano group, and a halogen atom. Examples of the halogen atom as a substituent include a fluorine atom, a chlorine atom, a bromine atom, and an iodine atom.

Specific examples of the aliphatic hydrocarbon group represented by R²¹⁰, R²²⁰, R²³⁰, R²⁴⁰, and R²⁵⁰ include a methyl group, an ethyl group, an n-propyl group, an isopropyl group, an n-butyl group, an isobutyl group, a sec-butyl group, a tert-butyl group, each isomer of a pentyl group, each isomer of a hexyl group, each isomer of a heptyl group, each isomer of an octyl group, each isomer of a nonyl group, each isomer of a decyl group, a cyclopentyl group, and a cyclohexyl group. Among these, an unsubstituted aliphatic hydrocarbon group having 1 to 12 carbon atoms is preferred, and a methyl group, an ethyl group, an n-propyl group, an isopropyl group, an n-butyl group, or an isobutyl group is preferred.

When an aromatic hydrocarbon group is selected for at least one of R²¹⁰, R²²⁰ R²³⁰, R²⁴⁰, and R²⁵⁰, the number of carbon atoms is preferably from 6 to 70, more preferably from 6 to 20, still more preferably from 6 to 12, and particularly preferably from 6 to 10.

Examples of the aromatic hydrocarbon group represented by R²¹⁰, R²²⁰, R²³⁰, R²⁴⁰, and R²⁵⁰ include an aryl group and aralkyl group, which may be unsubstituted or substituted. Examples of the substituent on these aromatic hydrocarbon groups include an aliphatic hydrocarbon group, a hydroxyl group, a cyano group, and a halogen atom. Examples of the aliphatic hydrocarbon group selected as the substituent on the aromatic hydrocarbon group represented by R²¹⁰, R²²⁰, R²³⁰, R²⁴⁰, and R²⁵⁰ include the same groups as those exemplified as the aliphatic hydrocarbon group represented by R²¹⁰, R²²⁰, R²³⁰, R²⁴⁰, and R²⁵⁰. Examples of the halogen atom as a substituent include a fluorine atom, a chlorine atom, a bromine atom, and an iodine atom.

Specific examples of the aromatic hydrocarbon group represented by R²¹⁰, R²²⁰ R²³⁰, R²⁴⁰, and R²⁵⁰ include a phenyl group, a naphthyl group, an anthryl group, a pyrenyl group, a phenanthryl group, an o-tolyl group, an m-tolyl group, a p-tolyl group, a benzyl group, and a phenethyl group.

When R²¹⁰ and R²²⁰ are bonded to each other to form a ring structure, a group formed by R²¹⁰ and R²²⁰ bonding to each other is a divalent organic group. The group formed by R²¹⁰ and R²²⁰ bonding to each other is preferably a substituted or unsubstituted divalent aliphatic hydrocarbon group having 1 to 70 carbon atoms or divalent aromatic hydrocarbon group having 6 to 70 carbon atoms, which may have an ether group, a carbonyl group, an ester group, an imino group (-NH-), an amide group, or an imide group.

In the group formed by R²¹⁰ and R²²⁰ bonding to each other, examples of the divalent aliphatic hydrocarbon group include an alkylene group such as a methylene group, an ethylene group, a propylene group, and a trimethylene group.

In the group formed by R²¹⁰ and R²²⁰ bonding to each other, examples of the divalent aromatic hydrocarbon group include an arylene group such as a phenylene group and a naphthylene group.

Regardless of whether or not R²¹⁰ and R²²⁰ are bonded to each other to form a ring structure, it is preferable that R²¹⁰ has a carbon atom at a position where it bonds to a nitrogen atom of the quinazolinedione structure. The carbon atom of R²¹⁰ at the position where R²¹⁰ bonds to a nitrogen atom of the quinazolinedione structure is preferably a primary carbon atom, a secondary carbon atom, a tertiary carbon atom, a carbon atom having aromaticity, or a carbonyl carbon atom.

Examples of preferred quinazolinedione compounds (II) include the following (II-1), (II-2), and (II-3).

In (II-1), in the above general formula (II), R²¹⁰ is a monovalent organic group, and R²²⁰, R²³⁰, R²⁴⁰, and R²⁵⁰ each independently represent a monovalent organic group or hydrogen.

In (II-1), examples of R²¹⁰ include a monovalent aliphatic hydrocarbon group having 1 to 12 carbon atoms and a monovalent aromatic hydrocarbon group having 6 to 12 carbon atoms, which may be substituted or unsubstituted. Specific examples of R²¹⁰ include a methyl group, an ethyl group, an n-propyl group, an isopropyl group, an n-butyl group, an isobutyl group, a sec-butyl group, a tert-butyl group, each isomer of a pentyl group, each isomer of a hexyl group, each isomer of a heptyl group, each isomer of an octyl group, each isomer of a nonyl group, each isomer of a decyl group, a cyclopentyl group, a cyclohexyl group, a phenyl group, a benzyl group, an o-tolyl group, an m-tolyl group, and a p-tolyl group. Among these, an unsubstituted aliphatic hydrocarbon group having 1 to 12 carbon atoms is preferred, and a methyl group, an ethyl group, an n-propyl group, an isopropyl group, an n-butyl group, or an isobutyl group is preferred.

In (II-1), the number of R²²⁰, R²³⁰, R²⁴⁰, and R²⁵⁰ serving as monovalent organic groups is an integer from 0 to 4, more preferably 0 or 1, and still more preferably 0.

When any one or more of R²²⁰, R²³⁰, R²⁴⁰, and R²⁵⁰ represent monovalent organic groups, they become substituents on the benzene ring in the quinazolinedione structure (hereinafter sometimes referred to as (a) "benzene ring substituent(s)"). Examples of the benzene ring substituent include an aliphatic hydrocarbon group having 1 to 12 carbon atoms or a monovalent aromatic hydrocarbon group having 6 to 12 carbon atoms, which may be substituted or unsubstituted. The benzene ring substituent may have an ether group, a carbonyl group, or an ester group.

When the benzene ring substituent is an aliphatic hydrocarbon group or an aromatic hydrocarbon group, examples thereof include an alkyl group having 1 to 12 carbon atoms, an aryl group having 6 to 12 carbon atoms, or an aralkyl group having 7 to 12 carbon atoms.

When the benzene ring substituent has an ether group, examples thereof include an alkoxy group having 1 to 12 carbon atoms, an aryloxy group having 6 to 12 carbon atoms, or an aralkyloxy group having 7 to 12 carbon atoms.

When the benzene ring substituent has a carbonyl group, examples thereof include an alkylcarbonyl group having 1 to 12 carbon atoms, an arylcarbonyl group having 6 to 12 carbon atoms, or an aralkylcarbonyl group having 7 to 12 carbon atoms.

When the benzene ring substituent has an ester group, examples thereof include an alkoxycarbonyl group or alkylcarbonyloxy group having 1 to 12 carbon atoms, an aryloxycarbonyl group or arylcarbonyloxy group having 6 to 12 carbon atoms, or an aralkyloxycarbonyl group or aralkylcarbonyloxy group having 7 to 12 carbon atoms.

Specific examples the benzene ring substituent include a methyl group, an ethyl group, an n-propyl group, an isopropyl group, an n-butyl group, an isobutyl group, a sec-butyl group, a tert-butyl group, each isomer of a pentyl group, each isomer of a hexyl group, each isomer of a heptyl group, each isomer of an octyl group, each isomer of a nonyl group, each isomer of a decyl group, a cyclopentyl group, a cyclohexyl group, a phenyl group, a benzyl group, an o-tolyl group, an m-tolyl group, and a p-tolyl group. Among these, an unsubstituted aliphatic hydrocarbon group having 1 to 12 carbon atoms is preferred, and a methyl group, an ethyl group, an n-propyl group, an isopropyl group, an n-butyl group, or an isobutyl group is preferred.

In (II-2), in the above general formula (II), R²¹⁰ and R²²⁰ are bonded to each other to form a ring structure via a carbon-carbon bond, and R²³⁰, R²⁴⁰, and R²¹⁰ each independently represent a monovalent organic group or hydrogen. The group formed by R²¹⁰ and R²²⁰ bonding to each other is an aliphatic hydrocarbon group having 1 to 12 carbon atoms or a monovalent aromatic hydrocarbon group having 6 to 12 carbon atoms, which may be substituted or unsubstituted.

Specific examples of the group formed by R²¹⁰ and R²²⁰ bonding to each other include a methylene group, an ethylene group, a propylene group, a trimethylene group, and a phenylene group.

The number of R²³⁰, R²⁴⁰, and R²⁵⁰ serving as benzene ring substituents is an integer from 0 to 3, more preferably 0 or 1, and still more preferably 0. Specific examples of R²³⁰, R²⁴⁰, and R²⁵⁰ in the case of serving as benzene ring substituents in (II-2) include the same as those exemplified as the benzene ring substituents in (II-1).

In (II-3), in the above general formula (II), R²¹⁰ and R²²⁰ are bonded to each other to form a ring structure via a carbon-carbon bond, a carbon-oxygen-carbon bond, or a carbon-nitrogen-carbon bond, and R²³⁰, R²⁴⁰, and R²⁵⁰ each independently represent a monovalent organic group or hydrogen. A group formed by R²¹⁰ and R²²⁰ bonding to each other is a divalent organic group represented by a general formula -R³¹¹-Z³¹¹-. With respect to the quinazolinedione structure, R³¹¹ is bonded to the same nitrogen atom to which R²¹⁰ is bonded, and Z³¹¹ is bonded to the same carbon atom to which R²²⁰ is bonded. Z³¹¹ is -O-, -NH-, or -C(=O)-. R³¹¹ is a divalent aliphatic hydrocarbon group having 1 to 12 carbon atoms or a divalent aromatic hydrocarbon group having 6 to 12 carbon atoms, which may be substituted or unsubstituted.

Specific examples of R³¹¹ include a methylene group, an ethylene group, a propylene group, a trimethylene group, and a phenylene group.

The number of R²³⁰, R²⁴⁰, and R²⁵⁰ serving as benzene ring substituents is an integer from 0 to 3, more preferably 0 or 1, and still more preferably 0. Specific examples of R²³⁰, R²⁴⁰, and R²⁵⁰ in the case of serving as benzene ring substituents in (II-3) include the same as those exemplified as the benzene ring substituents in (II-1).

Among the above general formulas (II), (11-1), (II-2), and (II-3), it is preferable that R²¹⁰, R²²⁰, R²³⁰, R²⁴⁰, and R²⁵⁰, as well as the group formed by R²¹⁰ and R²²⁰ bonding to each other, do not contain an olefinic or acetylenic unsaturated carbon-carbon bond, as the effects of reducing coloration are further enhanced. The hydrocarbon groups contained in R²¹⁰, R²²⁰, R²³⁰, R²⁴⁰, and R²⁵⁰, as well as the group formed by R²¹⁰ and R²²⁰ bonding to each other, are preferably saturated aliphatic hydrocarbon groups or aromatic hydrocarbon groups.

Further, from the viewpoint of thermal stability, it is preferable that R²¹⁰, R²²⁰ R²³⁰, R²⁴⁰, and R²⁵⁰, as well as the group formed by R²¹⁰ and R²²⁰ bonding to each other, have a structure constituted solely of carbon atoms and hydrogen atoms, excluding Z³¹¹ in (II-3).

In (11-1), it is preferable that R²¹⁰ is a monovalent aliphatic hydrocarbon group, three or more of R²²⁰, R²³⁰, R²⁴⁰, and R²⁵⁰ are hydrogen, and one or less of R²²⁰ R²³⁰, R²⁴⁰, and R²⁵⁰ is an unsubstituted aliphatic hydrocarbon group having 1 to 12 carbon atoms or aromatic hydrocarbon group having 6 to 12 carbon atoms.

In a more preferred (II-1), it is preferable that R²¹⁰ is a monovalent aliphatic hydrocarbon group having 1 to 12 carbon atoms, three or more of R²²⁰, R²³⁰, R²⁴⁰, and R²⁵⁰ are hydrogen, and one or less of R²²⁰, R²³⁰, R²⁴⁰, and R²⁵⁰ is a methyl group or an ethyl group.

In (11-2), it is preferable that the group formed by R²¹⁰ and R²²⁰ bonding to each other is a divalent aliphatic hydrocarbon group, two or more of R²³⁰, R²⁴⁰, and R²⁵⁰ are hydrogen, and one or less of R²³⁰, R²⁴⁰, and R²⁵⁰ is an unsubstituted aliphatic hydrocarbon group having 1 to 12 carbon atoms or aromatic hydrocarbon group having 6 to 12 carbon atoms.

In a more preferred form of (II-2), it is preferable that the group formed by R²¹⁰ and R²²⁰ bonding to each other is a divalent aliphatic hydrocarbon group having 1 to 12 carbon atoms, two or more of R²³⁰, R²⁴⁰, and R²⁵⁰ are hydrogen, and one or less of R²³⁰, R²⁴⁰, and R²⁵⁰ is a methyl group or an ethyl group.

In (II-3), it is preferable that R³¹⁰ is a divalent aliphatic hydrocarbon group, two or more of R²³⁰, R²⁴⁰, and R²⁵⁰ are hydrogen, and one or less of R²³⁰, R²⁴⁰, and R²⁵⁰ is an unsubstituted aliphatic hydrocarbon group having 1 to 12 carbon atoms or aromatic hydrocarbon group having 6 to 12 carbon atoms.

In a more preferred form of (II-3), it is preferable that R³¹⁰ is a divalent aliphatic hydrocarbon group having 1 to 12 carbon atoms, two or more of R²³⁰, R²⁴⁰, and R²⁵⁰ are hydrogen, and one or less of R²³⁰, R²⁴⁰, and R²⁵⁰ is a methyl group or an ethyl group.

### [Method for producing quinazolinedione compound (II)]

The quinazolinedione compound (II) can be synthesized using a known production method. Examples of the method for synthesizing the quinazolinedione compound (II) include a method for reacting an aniline compound with a carbonic acid derivative. The carbonic acid derivative used in the synthesis of quinazolinedione compound (II) may be one type or two or more types.

The aniline compound used in the synthesis of quinazolinedione compound (II) has an unsubstituted or monosubstituted nitrogen atom, and at least one of the carbon atoms in the ortho position with respect to the nitrogen atom of the benzene ring is unsubstituted. The quinazolinedione compound (II) can be obtained by forming a - C(=O)-NH-C(=O)- group between the nitrogen atom of the aniline compound and one of the carbon atoms in the ortho position.

Examples of the above-mentioned aniline compound include an unsubstituted aniline or a substituted aniline having an organic group identical to at least one of R²¹⁰, R²²⁰, R²³⁰, R²⁴⁰, and R²⁵⁰ of the quinazolinedione compound (II) as a substituent. R²¹⁰ and R²²⁰ may be bonded to each other to form a ring structure via a carbon-carbon bond, a carbon-oxygen-carbon bond, or a carbon-nitrogen-carbon bond.

The above carbonic acid derivative is not particularly limited, but due to its ease of availability, a compound represented by the following general formula (A) (hereinafter sometimes referred to as a "carbonic acid derivative (A)") is preferably used.

R⁶¹-C(=O)-R⁶² (A)

In the above general formula (A), R⁶¹ and R⁶² each independently represent an amino group, an alkoxy group having 1 to 20 carbon atoms or aryloxy group having 6 to 20 carbon atoms, which may be substituted or unsubstituted, or an alkylamino group having 1 to 20 carbon atoms or arylamino group having 6 to 20 carbon atoms, which may be substituted or unsubstituted.

R⁶¹ and R⁶² may be the same as or different from each other. The alkylamino group may be a monoalkylamino group or a dialkylamino group. The arylamino group may be a monoarylamino group, a diarylamino group, or an alkyl(aryl)amino group.

Examples of the alkoxy group having 1 to 20 carbon atoms represented by R⁶¹ and R⁶² include a methoxy group, an ethoxy group, each isomer of a propoxy group, each isomer of a butoxy group, and each isomer of a hexyloxy group.

Examples of the aryloxy group having 6 to 20 carbon atoms represented by R⁶¹ and R⁶² include a phenoxy group and a naphthyloxy group.

Examples of the alkylamino group having 1 to 20 carbon atoms represented by R⁶¹ and R⁶² include a methylamino group, an ethylamino group, each isomer of a propylamino group, each isomer of a butylamino group, and each isomer of a hexylamino group.

Examples of the arylamino group having 6 to 20 carbon atoms represented by R⁶¹ and R⁶² include a phenylamino group and a naphthylamino group.

Examples of the substituent which an alkoxy group, aryloxy group, alkylamino group, and arylamino group may have include an alkyl group and an alkoxy group.

Among these, it is preferable that R⁶¹ and R⁶² each independently represent an amino group, a substituted or unsubstituted aryloxy group having 6 to 20 carbon atoms, or a substituted or unsubstituted arylamino group having 6 to 20 carbon atoms.

Preferred examples of the carbonic acid derivative (A) include a urea compound, a carbamic acid ester, and a carbonic acid ester.

### [Urea compound]

Examples of the urea compound include a compound represented by the above general formula (A) in which R⁶¹ and R⁶² each independently represent a substituted or unsubstituted alkylamino group or arylamino group. Among the urea compounds, compounds represented by the following general formula (A-1) are preferred.

R⁶¹¹-(R⁶¹²-)N-C(=O)-N(-R⁶¹³)-R⁶¹⁴ (A-1)

In the above general formula (A-1), R⁶¹¹, R⁶¹², R⁶¹³, and R⁶¹⁴ each independently represent an alkyl group having 1 to 20 carbon atoms, an aralkyl group having 7 to 20 carbon atoms, an aryl group having 6 to 20 carbon atoms, or a hydrogen atom. The total number of carbon atoms constituting R⁶¹¹ and R⁶¹² is an integer from 0 to 20, and the total number of carbon atoms constituting R⁶¹³ and R⁶¹⁴ is an integer from 0 to 20.

Examples of the urea compound include an unsubstituted urea compound, a monosubstituted urea compound, an N,N-disubstituted urea compounds, an N,N'-disubstituted urea compound, a trisubstituted urea compound, and a tetrasubstituted urea compound.

### [Carbamic acid ester]

Examples of the carbamic acid ester include a compound represented by the above general formula (A) in which one of R⁶¹ and R⁶² is an amino group, or a substituted or unsubstituted alkylamino group or arylamino group, and the other of R⁶¹ and R⁶² is a substituted or unsubstituted alkoxy group or aryloxy group. Among the carbamic acid esters, compounds represented by the following general formula (A-2) are preferred.

R⁶²²-(R⁶²¹-)N-C(=O)-O-R⁶²¹ (A-2)

In the above general formula (A-2), R⁶²¹ is an alkyl group having 1 to 20 carbon atoms, an aralkyl group having 7 to 20 carbon atoms, or an aryl group having 6 to 20 carbon atoms. R⁶²² and R⁶²³ each independently represent a hydrogen atom, an alkyl group having 1 to 20 carbon atoms, an aralkyl group having 7 to 20 carbon atoms, or an aryl group having 6 to 20 carbon atoms.

Examples of the carbamic acid ester include an N-unsubstituted carbamic acid ester, an N-monosubstituted carbamic acid ester, and an N,N-disubstituted carbamic acid ester.

### [Carbonic acid ester]

Examples of the carbonic acid ester include a compound represented by the above general formula (A) in which R⁶¹ and R⁶² each independently represent a substituted or unsubstituted alkoxy group or aryloxy group. Among the carbonic acid esters, compounds represented by the following general formula (A-3) are preferred.

R⁶¹¹-O-C(=O)-O-R⁶³² (A-3)

In the above general formula (A-3), R⁶³¹ and R⁶³² each independently represent an aralkyl group having 7 to 20 carbon atoms, an alkyl group having 1 to 20 carbon atoms, or an aryl group having 6 to 20 carbon atoms.

The quinazolinedione compound (II) has active hydrogen and provides excellent storage stability and coloration inhibition for the polyisocyanate composition 2. The quinazolinedione compound (II) inhibits the formation of a 1-nylon structure, which is a "compound having UV absorption in a region of a decamer or higher isocyanate in GPC," as described later, and acts as a stabilizer during storage.

Because the quinazolinedione compound (II) has a benzene ring in its quinazolinedione structure, the surrounding of the active hydrogen contained in the quinazolinedione structure is bulky, and an addition reaction with an isocyanate group either does not occur or occurs only slightly. Further, because the benzene ring has electron withdrawing properties, an anion generated when the active hydrogen contained in the quinazolinedione structure is abstracted by a base is stabilized, making it possible to inhibit the formation of a 1-nylon structure.

Although it is preferable to increase the content of the quinazolinedione compound (II) in order to improve the storage stability and coloration inhibition of the polyisocyanate composition 2, when the content of the quinazolinedione compound (II) is too high, the production cost, applications, and the like of the polyisocyanate composition 2 may be adversely affected.

The polyisocyanate composition 2 preferably contains 1.0 ppm by mass or more and 5.0 × 10⁴ ppm by mass or less of quinazolinedione compound (II), and more preferably 1.0 ppm by mass or more and 1.0 × 10⁴ ppm by mass or less of quinazolinedione compound (II), based on the total mass of the polyisocyanate compounds.

The polyisocyanate composition 2 of the present embodiment can contain an optional component other than the quinazolinedione compound (II) and the polyisocyanate compound, depending on the application, purpose, and the like.

The polyisocyanate composition 2 of the present embodiment can be used as a material for synthesizing polyurethane, a curing agent, and the like.

The polyisocyanate composition 2 of the present embodiment can be utilized in a wide range of fields, including flexible foams, rigid foams, elastomers, adhesives, coating materials, and binders.

### [Compound having UV absorption in a region of a decamer or higher isocyanate in GPC]

In gel permeation chromatography (hereinafter sometimes referred to as "GPC"), compounds having UV absorption in a region of a decamer or higher isocyanate in GPC are preferably compounds having a 1-nylon structure represented by the following general formula (W) as their main skeletons.

-[N(-R^{W})-C(=O)]w- (W)

In the above general formula (W), R^{W} represents a residue obtained by removing one isocyanate group from a polyisocyanate compound, and W represents an integer of 1 or more. Further, a terminal group is not shown.

An isocyanate constituting the compound having UV absorption in a region of a decamer or higher isocyanate in a measurement spectrum by GPC may be the same isocyanate as or a different isocyanate from the isocyanate compound constituting the isocyanate composition of the present embodiment.

This compound is defined by GPC measurement. More specifically, when polystyrene is used as a reference material of molecular weight in GPC using tetrahydrofuran as a developing solvent, it exhibits a peak with UV absorption at a wavelength of 254 nm in the region of a decamer or higher isocyanate.

In the measurement spectrum by GPC, the concentration of the compound having UV absorption in the region of a decamer or higher isocyanate can be determined from a value calculated by (B)/(A), where (A) is an area of the peak corresponding to a bifunctional or higher isocyanate in UV absorption (wavelength: 210 nm) measured by a PDA detector, and (B) is an area of the peak corresponding to a compound having UV absorption (wavelength: 254 nm) in the region of a decamer or higher isocyanate.

### <<Polyisocyanate composition 3>>

The polyisocyanate composition 3 of the present embodiment contains a trisubstituted urea compound (III) and a polyisocyanate compound.

### [Compound (III)]

The compound (III) is represented by the following general formula (III). Hereinafter, the compound represented by the following general formula (III) may be referred to as a "trisubstituted urea compound (III)." 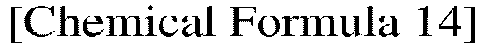 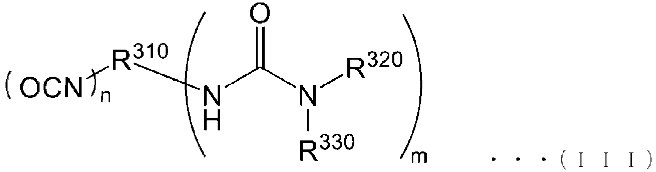 (In the general formula (III), R³¹⁰ is an (n + m )-valent organic group, n is an integer from 0 to 12, m is an integer from 1 to 12, n + m is an integer of 13 or less, and R³²⁰ and R³³⁰ each independently represent a monovalent organic group; R³²⁰ and R³³⁰ may be bonded to each other to form a ring structure via a carbon-carbon bond, a carbon-oxygen-carbon bond, or a carbon-nitrogen-carbon bond; and at least one of R³²⁰ and R³³⁰ has an aromatic group.)

The trisubstituted urea compound (III) has a structure in which an (OCN)ₙ-R³¹⁰-group, an R³²⁰ group, and an R³³⁰ group are bonded to a urea molecule represented by H₂N-CO-NH₂ as substituents (hereinafter sometimes referred to as a "trisubstituted urea structure"). The trisubstituted urea structure is a monovalent group represented by -NH-C(=O)-N(-R³³⁰)-R³²⁰ in the above general formula (III).

The trisubstituted urea structure contains an active hydrogen atom in the -NH-C(=O)-N< group. However, the -NH-C(=O)-N< group contained in the trisubstituted urea structure may exhibit tautomerism represented by -N=C(-OH)-N<.

The inventors of the present invention and others discovered that the problems of thermal denaturation and coloration can be solved by producing a blocked isocyanate in the presence of a specific amine compound having an aromatic group, thereby leading to completion of the present invention.

Conventionally, compounds having an amino group bonded to a carbon atom having aromaticity have been known as compounds that are easily oxidized and cause coloration. Therefore, although compounds having an amino group bonded to a carbon atom having aromaticity have been used at times in products where coloration is not an issue, such as rubber degradation inhibitors, they have not been used in products where coloration is an issue.

On the other hand, according to the polyisocyanate composition of the present embodiment, it has now been surprisingly discovered for the first time that even when using a specific amine compound having an aromatic group, far from causing the problem of coloration, an exceptionally remarkable effect of improving (inhibiting) thermal denaturation and coloration is exhibited.

### [R³¹⁰]

In the above general formula (III), R³¹⁰ is an (n + m)-valent organic group, where n is an integer from 0 to 12, m is an integer from 1 to 12, and n + m is an integer of 13 or less.

n represents the number of isocyanate groups (OCN-) bonded to R³¹⁰. m represents the number of trisubstituted urea structures bonded to R³¹⁰. Since n is an integer equal to or greater than 0 and m is an integer equal to or greater than 1, n + m is an integer equal to or greater than 1.

The trisubstituted urea compound (III) is a compound having n isocyanate groups. For this reason, R³¹⁰ is an organic group that does not contain an isocyanate group.

The trisubstituted urea compound (III) is a compound having m trisubstituted urea structures. For this reason, R³¹⁰ is an organic group that does not contain a trisubstituted urea structure.

The trisubstituted urea compound (III) may be a compound that does not have a blocked isocyanate group, except in cases where the trisubstituted urea structure corresponds to a blocked isocyanate group. In this case, R³¹⁰ is an organic group that does not contain a blocked isocyanate group.

The blocked isocyanate group is a group shown on the left side of the following reaction formula (K), and as shown on the right side of the reaction formula (K), it is a group that can thermally dissociate into an isocyanate group and a blocking agent represented by a general formula BL-H.

-NH-C(=O)-BL → -N=C=O + BL-H (K)

In the above reaction formula (K), the blocking agent represented by BL-H is a compound having active hydrogen. Examples of the blocking agent include a phenol-based blocking agent, an alcohol-based blocking agent, a thiol-based blocking agent, an amine-based blocking agent, an ammonia-based blocking agent, an oxime-based blocking agent, a hydroxylamine-based blocking agent, and an active methylene-based blocking agent.

Examples of the phenol-based blocking agent include an organic compound that has an -OH group bonded to a carbon atom having aromaticity.

Examples of the alcohol-based blocking agent include an organic compound that has an -OH group bonded to a carbon atom having no aromaticity.

Examples of the thiol-based blocking agent include an organic compound having an -SH group.

Examples of the amine-based blocking agent include an organic compound having an -NH₂ group or an -NH- group. A triazole-based compound, a pyrazole-based compound, and the like are also included in the amine-based blocking agent having an - NH- group.

Examples of the ammonia-based blocking agent include ammonia.

Examples of the oxime-based blocking agent include an organic compound having an =N-OH group bonded to one carbon atom.

Examples of the hydroxylamine-based blocking agent include an organic compound having an >N-OH group bonded to two carbon atoms.

The active methylene-based blocking agent is an organic compound containing an atomic group such as a -C(=O)-C(-H)₂-C(=O)- group, and examples thereof include an acetoacetic acid ester-based compound, a malonic acid diester-based compound, and acetylacetone.

The trisubstituted urea compound (III) may be a compound that does not have a unit constituting an isocyanate polymer. In this case, R³¹⁰ is an organic group that does not contain a unit constituting an isocyanate polymer.

Among units containing a nitrogen atom derived from one or more isocyanate groups, the unit constituting an isocyanate polymer refer to an isocyanurate group, a biuret group, an iminooxadiazinedione group, a ureylene group (-NH-C(=O)-NH-), an allophanate group, a uretdione group, and a urethane group.

R³¹⁰ is preferably a substituted or unsubstituted (n + m)-valent aliphatic hydrocarbon group having 1 to 70 carbon atoms or (n + m)-valent aromatic hydrocarbon group having 6 to 70 carbon atoms, which may have an ether group, a carbonyl group, an ester group, an imino group (-NH-), an amide group, or an imide group.

Examples of the monovalent aliphatic hydrocarbon group represented by R³¹⁰ include a substituted or unsubstituted alkyl group and cycloalkyl group.

Examples of the divalent aliphatic hydrocarbon group represented by R³¹⁰ include a substituted or unsubstituted alkylene group and cycloalkylene group.

Examples of the trivalent aliphatic hydrocarbon group represented by R³¹⁰ include a substituted or unsubstituted alkanetriyl group and cycloalkanetriyl group.

Examples of the tetravalent aliphatic hydrocarbon group represented by R³¹⁰ include a substituted or unsubstituted alkanetetrayl group and cycloalkanetetrayl group.

Examples of the pentavalent aliphatic hydrocarbon group represented by R³¹⁰ include a substituted or unsubstituted alkanepentyl group and cycloalkanepentyl group.

Examples of the hexavalent aliphatic hydrocarbon group represented by R³¹⁰ include a substituted or unsubstituted alkanehexyl group and cycloalkanehexyl group.

Examples of the heptavalent aliphatic hydrocarbon group represented by R³¹⁰ include a substituted or unsubstituted alkaneheptyl group and cycloalkaneheptyl group.

Examples of the octavalent aliphatic hydrocarbon group represented by R³¹⁰ include a substituted or unsubstituted alkaneoctyl group and cycloalkaneoctyl group.

Examples of the nonavalent aliphatic hydrocarbon group represented by R³¹⁰ include a substituted or unsubstituted alkanenonyl group and cycloalkanenonyl group.

Examples of the decavalent aliphatic hydrocarbon group represented by R³¹⁰ include a substituted or unsubstituted alkanedecyl group and cycloalkanedecyl group.

Examples of the undecavalent aliphatic hydrocarbon group represented by R³¹⁰ include a substituted or unsubstituted alkaneundecayl group and cycloalkaneundecayl group.

Examples of the dodecavalent aliphatic hydrocarbon group represented by R³¹⁰ include a substituted or unsubstituted alkanedodecyl group and cycloalkanedodecyl group.

Examples of the tridecavalent aliphatic hydrocarbon group represented by R³¹⁰ include a substituted or unsubstituted alkanetridecyl group and cycloalkanetridecyl group.

When an aliphatic hydrocarbon group is selected for R³¹⁰, the number of carbon atoms is preferably from 1 to 70, more preferably from 1 to 20, still more preferably from 1 to 12, and particularly preferably from 1 to 10.

Examples of the substituent on the aliphatic hydrocarbon group represented by R³¹⁰ include a hydroxyl group, a cyano group, and a halogen atom. Examples of the halogen atom as a substituent include a fluorine atom, a chlorine atom, a bromine atom, and an iodine atom. The number of substituents is not particularly limited, but is preferably from 0 to 10.

Specific examples of the aliphatic hydrocarbon group represented by R³¹⁰ include a methyl group, an ethyl group, an n-propyl group, an isopropyl group, an n-butyl group, an isobutyl group, a sec-butyl group, a tert-butyl group, each isomer of a pentyl group, each isomer of a hexyl group, each isomer of a heptyl group, each isomer of an octyl group, each isomer of a nonyl group, each isomer of a decyl group, a cyclopentyl group, and a cyclohexyl group. Among these, an unsubstituted aliphatic hydrocarbon group having 1 to 12 carbon atoms is preferred, and a methyl group, an ethyl group, an n-propyl group, an isopropyl group, an n-butyl group, or an isobutyl group is preferred.

Examples of the monovalent aromatic hydrocarbon group represented by R³¹⁰ include a substituted or unsubstituted aryl group.

Examples of the divalent aromatic hydrocarbon group represented by R³¹⁰ include a substituted or unsubstituted arylene group.

Examples of the trivalent aromatic hydrocarbon group represented by R³¹⁰ include a substituted or unsubstituted arenetriyl group.

Examples of the tetravalent aromatic hydrocarbon group represented by R³¹⁰ include a substituted or unsubstituted arenetetrayl group.

Examples of the pentavalent aromatic hydrocarbon group represented by R³¹⁰ include a substituted or unsubstituted arenepentyl group.

Examples of the hexavalent aromatic hydrocarbon group represented by R³¹⁰ include a substituted or unsubstituted arenehexyl group.

Examples of the heptavalent aromatic hydrocarbon group represented by R³¹⁰ include a substituted or unsubstituted areneheptyl group.

Examples of the octavalent aromatic hydrocarbon group represented by R³¹⁰ include a substituted or unsubstituted areneoctyl group.

Examples of the nonavalent aromatic hydrocarbon group represented by R³¹⁰ include a substituted or unsubstituted arenenonyl group.

Examples of the decavalent aromatic hydrocarbon group represented by R³¹⁰ include a substituted or unsubstituted arenedecyl group.

Examples of the undecavalent aromatic hydrocarbon group represented by R³¹⁰ include a substituted or unsubstituted areneundecyl group.

Examples of the dodecavalent aromatic hydrocarbon group represented by R³¹⁰ include a substituted or unsubstituted arenedodecyl group.

Examples of the tridecavalent aromatic hydrocarbon group represented by R³¹⁰ include a substituted or unsubstituted arenetridecyl group.

When an aromatic hydrocarbon group is selected for R³¹⁰, the number of carbon atoms is preferably from 6 to 70, more preferably from 6 to 20, still more preferably from 6 to 12, and particularly preferably from 6 to 10.

Examples of the aryl group represented by R³¹⁰ include a phenyl group, a naphthyl group, an anthryl group, a pyrenyl group, and a phenanthryl group.

Examples of the arylene group represented by R³¹⁰ include a phenylene group, a naphthylene group, an anthrylene group, a pyrenylene group, and a phenanthrylene group.

Examples of an arene ring contained in the trivalent to tridecavalent aromatic hydrocarbon group represented by R³¹⁰ include an aromatic hydrocarbon ring such as a benzene ring, a naphthalene ring, an anthracene ring, a pyrene ring, and a phenanthrene ring.

Examples of the substituent on the aromatic hydrocarbon group represented by R³¹⁰ include an aliphatic hydrocarbon group, a hydroxyl group, a cyano group, and a halogen atom. Examples of the aliphatic hydrocarbon group selected as the substituent on the aromatic hydrocarbon group represented by R³¹⁰ include the same groups as those exemplified as the aliphatic hydrocarbon group represented by R³¹⁰. Examples of the halogen atom as a substituent include a fluorine atom, a chlorine atom, a bromine atom, and an iodine atom. The number of substituents is not particularly limited, but is preferably from 0 to 10.

It is preferable that R³¹⁰ has a carbon atom at a position where it bonds to an isocyanate group or a nitrogen atom of the trisubstituted urea structure. The carbon atom of R³¹⁰ at the position where R³¹⁰ bonds to an isocyanate group or a nitrogen atom of the trisubstituted urea structure is preferably a primary carbon atom, a secondary carbon atom, a tertiary carbon atom, a carbon atom having aromaticity, or a carbonyl carbon atom.

The trisubstituted urea compound (III) may be a compound represented by the following general formula (III-a).

(Z³¹¹-)ₖ₁-R⁴¹⁰-L³¹¹-R⁴²⁰-(-Z³¹²)ₖ₂ ···(III-a)

In the above general formula (III-a), R⁴¹⁰ is a (k1 + 1)-valent cyclic aliphatic hydrocarbon group or aromatic hydrocarbon group, L³¹¹ is a single bond or a divalent acyclic aliphatic hydrocarbon group, and R⁴²⁰ is a (k2 + 1)-valent cyclic aliphatic hydrocarbon group or aromatic hydrocarbon group. Z³¹¹ and Z³¹² each independently represent an isocyanate group or a trisubstituted urea structure. The number of isocyanate groups represented by Z³¹¹ and Z³¹² is within a range of n in the general formula (III), the number of trisubstituted urea structures represented by Z³¹¹ and Z³¹² is within a range of m in the general formula (III), k1 + k2 is within a range of n + m, and each of k1 and k2 is an integer of 1 or more.

The -R⁴¹⁰-L³¹¹-R⁴²⁰- group in the general formula (III-a) corresponds to R³¹⁰ in the general formula (III). Examples of the cyclic or acyclic aliphatic hydrocarbon group or aromatic hydrocarbon group represented by R⁴¹⁰, R⁴²⁰, and L³¹¹ include the same groups as those exemplified as the aliphatic hydrocarbon group or aromatic hydrocarbon group represented by R³¹⁰ in the general formula (III).

The trisubstituted urea compound (III) may be a compound represented by the following general formula (III-b).

(Z³¹³-)ₖ₃-R³¹³-L³¹³-R³¹⁴-(-Z³¹⁴)ₖ₄ ···(III-b)

In the above general formula (III-b), R³¹³ is a (k3 + 1)-valent aliphatic hydrocarbon group or aromatic hydrocarbon group, L³¹³ is an ether group, a carbonyl group, an ester group, an imino group (-NH-), an amide group, or an imide group, and R³¹⁴ is a (k4 + 1)-valent aliphatic hydrocarbon group or aromatic hydrocarbon group. Z³¹³ and Z³¹⁴ each independently represent an isocyanate group or a trisubstituted urea structure. The number of isocyanate groups represented by Z³¹³ and Z³¹⁴ is within a range of n in the general formula (III), the number of trisubstituted urea structures represented by Z³¹³ and Z³¹⁴ is within a range of m in the general formula (III), k3 + k4 is within a range of n + m, and each of k3 and k4 is an integer of 1 or more.

The -R³¹³-L³¹³-R³¹⁴- group in the general formula (III-b) corresponds to R³¹⁰ in the general formula (III). Examples of the aliphatic hydrocarbon group or aromatic hydrocarbon group represented by R³¹³ and R³¹⁴ include the same groups as those exemplified as the aliphatic hydrocarbon group or aromatic hydrocarbon group represented by R³¹⁰ in the general formula (III).

Examples of trisubstituted urea compounds (III) in which R³¹⁰ is specifically specified include compounds represented by the following general formulas (1) to (10).

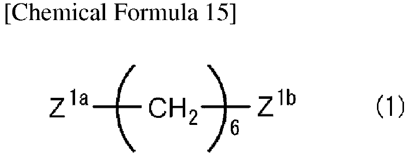

In the above general formula (1), Z^{1a} and Z^{1b} each independently represent an isocyanate group or a trisubstituted urea structure. The number of isocyanate groups represented by Z^{1a} and Z^{1b} is 0 or 1, the number of trisubstituted urea structures represented by z^{1a} and Z^{1b} is 1 or 2, and the total number of isocyanate groups or trisubstituted urea structures represented by Z^{1a} and Z^{1b} is 2.

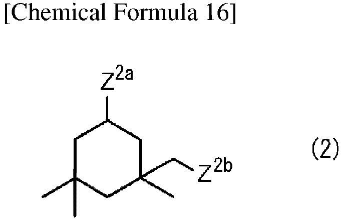

In the above general formula (2), Z^{2a} and Z^{2b} each independently represent an isocyanate group or a trisubstituted urea structure. The number of isocyanate groups represented by Z^{2a} and Z^{2b} is 0 or 1, the number of trisubstituted urea structures represented by Z^{2a} and Z^{2b} is 1 or 2, and the total number of isocyanate groups or trisubstituted urea structures represented by Z^{2a} and Z^{2b} is 2.

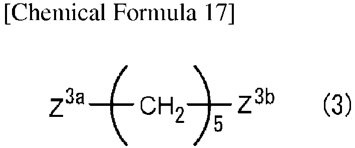

In the above general formula (3), Z^{3a} and Z^{3b} each independently represent an isocyanate group or a trisubstituted urea structure. The number of isocyanate groups represented by Z^{3a} and Z^{3b} is 0 or 1, the number of trisubstituted urea structures represented by Z^{3a} and Z^{3b} is 1 or 2, and the total number of isocyanate groups or trisubstituted urea structures represented by Z^{3a} and Z^{3b} is 2.

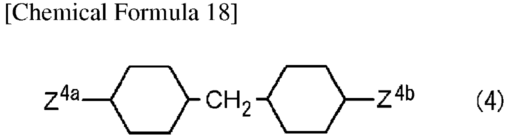

In the above general formula (4), z^{4a} and Z^{4b} each independently represent an isocyanate group or a trisubstituted urea structure. The number of isocyanate groups represented by Z^{4a} and Z^{4b} is 0 or 1, the number of trisubstituted urea structures represented by Z^{4a} and Z^{4b} is 1 or 2, and the total number of isocyanate groups or trisubstituted urea structures represented by Z^{4a} and Z^{4b} is 2. The compound represented by the general formula (4) may be an isomer mixture.

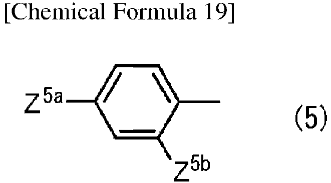

In the above general formula (5), Z^{5a} and Z^{5b} each independently represent an isocyanate group or a trisubstituted urea structure. The number of isocyanate groups represented by Z^{5a} and Z^{5b} is 0 or 1, the number of trisubstituted urea structures represented by Z^{5a} and Z^{5b} is 1 or 2, and the total number of isocyanate groups or trisubstituted urea structures represented by Z^{5a} and Z^{5b} is 2.

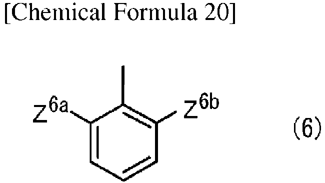

In the above general formula (6), Z^{6a} and Z^{6b} each independently represent an isocyanate group or a trisubstituted urea structure. The number of isocyanate groups represented by Z^{6a} and Z^{6b}, is 0 or 1, the number of trisubstituted urea structures represented by Z^{6a} and Z^{6b} is 1 or 2, and the total number of isocyanate groups or trisubstituted urea structures represented by Z^{6a} and Z^{6b} is 2.

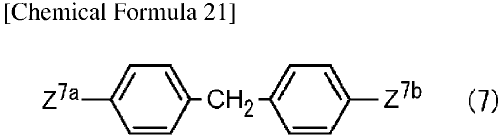

In the above general formula (7), Z^{7a} and Z^{7b} each independently represent an isocyanate group or a trisubstituted urea structure. The number of isocyanate groups represented by Z^{7a} and Z^{7b} is 0 or 1, the number of trisubstituted urea structures represented by Z^{7a} and Z^{7b} is 1 or 2, and the total number of isocyanate groups or trisubstituted urea structures represented by Z^{7a} and Z^{7b} is 2.

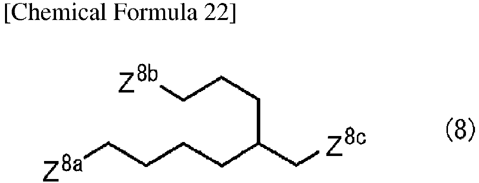

In the above general formula (8), Z^{8a}, Z^{8b}, and Z^{8c} each independently represent an isocyanate group or a trisubstituted urea structure. The number of isocyanate groups represented by Z^{8a}, Z^{8b}, and Z^{8c} is 0, 1, or 2, the number of trisubstituted urea structures represented by Z^{8a}, Z^{8b}, and Z^{8c} is 1, 2, or 3, and the total number of isocyanate groups or trisubstituted urea structures represented by Z^{8a}, Z^{8b}, and Z^{8c} is 3.

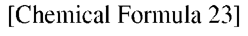

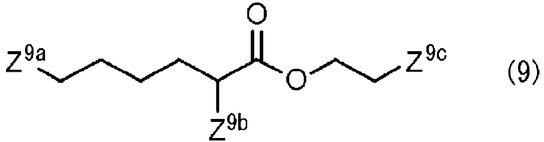

In the above general formula (9), Z^{9a}, Z^{9b}, and Z^{9c} each independently represent an isocyanate group or a trisubstituted urea structure. The number of isocyanate groups represented by Z^{9a}, Z^{9b}, and Z^{9c} is 0, 1, or 2, the number of trisubstituted urea structures represented by Z^{9a}, Z^{9b} , and Z^{9c} is 1, 2, or 3, and the total number of isocyanate groups or trisubstituted urea structures represented by Z^{9a}, Z^{9b}, and Z^{9c} is 3.

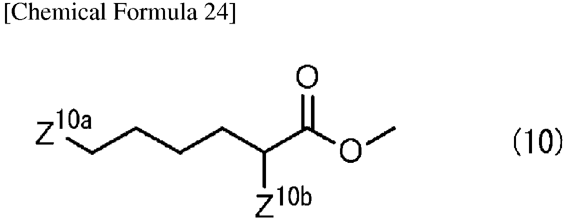

In the above general formula (10), Z^{10a} and Z^{10b} each independently represent an isocyanate group or a trisubstituted urea structure. The number of isocyanate groups represented by Z^{10a} and Z^{10b} is 0 or 1, the number of trisubstituted urea structures represented by Z^{10a} and Z^{10b} is 1 or 2, and the total number of isocyanate groups or trisubstituted urea structures represented by Z^{10a} and Z^{10b} is 2.

### [R¹²⁰ and R³¹⁰]

In the above general formula (III), R² and R³ each independently represent a monovalent organic group. R³²⁰ and R³³⁰ may be bonded to each other to form a ring structure via a carbon-carbon bond, a carbon-oxygen-carbon bond, or a carbon-nitrogen-carbon bond. At least one of R³²⁰ and R³³⁰ has an aromatic group.

In particular, R³²⁰ and R³³⁰ preferably each independently represent a substituted or unsubstituted monovalent aliphatic hydrocarbon group having 1 to 70 carbon atoms or monovalent aromatic hydrocarbon group having 6 to 70 carbon atoms, which may have an ether group, a carbonyl group, an ester group, an imino group (-NH-), an amide group, or an imide group.

When an aliphatic hydrocarbon group is selected for at least one of R³²⁰ and R³³⁰, the number of carbon atoms is preferably from 1 to 70, more preferably from 1 to 20, still more preferably from 1 to 12, and particularly preferably from 1 to 10.

Examples of the aliphatic hydrocarbon group represented by R³²⁰ and R³³⁰ include a linear alkyl group, branched alkyl group, and cycloalkyl group, which may be unsubstituted or substituted. Examples of the substituent on these aliphatic hydrocarbon groups include a hydroxyl group, a cyano group, and a halogen atom. Examples of the halogen atom as a substituent include a fluorine atom, a chlorine atom, a bromine atom, and an iodine atom.

Specific examples of the aliphatic hydrocarbon group represented by R³²⁰ and R³³⁰ include a methyl group, an ethyl group, an n-propyl group, an isopropyl group, an n-butyl group, an isobutyl group, a sec-butyl group, a tert-butyl group, each isomer of a pentyl group, each isomer of a hexyl group, each isomer of a heptyl group, each isomer of an octyl group, each isomer of a nonyl group, each isomer of a decyl group, a cyclopentyl group, and a cyclohexyl group. Among these, an unsubstituted aliphatic hydrocarbon group having 1 to 12 carbon atoms is preferred, and a methyl group, an ethyl group, an n-propyl group, an isopropyl group, an n-butyl group, or an isobutyl group is preferred.

When an aromatic hydrocarbon group is selected for at least one of R³²⁰ and R³³⁰, the number of carbon atoms is preferably from 6 to 70, more preferably from 6 to 20, still more preferably from 6 to 12, and particularly preferably from 6 to 10.

Examples of the aromatic hydrocarbon group represented by R³²⁰ and R³³⁰ include an aryl group and aralkyl group, which may be unsubstituted or substituted. Examples of the substituent on these aromatic hydrocarbon groups include an aliphatic hydrocarbon group, a hydroxyl group, a cyano group, and a halogen atom. Examples of the aliphatic hydrocarbon group selected as the substituent on the aromatic hydrocarbon group represented by R³²⁰ and R¹³⁰ include the same groups as those exemplified as the aliphatic hydrocarbon group represented by R³²⁰ and R³³⁰. Examples of the halogen atom as a substituent include a fluorine atom, a chlorine atom, a bromine atom, and an iodine atom.

Specific examples of the aromatic hydrocarbon group represented by R³²⁰ and R³³⁰ include a phenyl group, a naphthyl group, an anthryl group, a pyrenyl group, a phenanthryl group, an o-tolyl group, an m-tolyl group, a p-tolyl group, a benzyl group, and a phenethyl group.

When R³²⁰ and R³³⁰ are bonded to each other to form a ring structure, a group formed by R³²⁰ and R¹¹⁰ bonding to each other is a divalent organic group. The group formed by R³²⁰ and R³³⁰ bonding to each other is a substituted or unsubstituted divalent group having 7 to 70 carbon atoms, which may have an ether group, a carbonyl group, an ester group, an imino group (-NH-), an amide group, or an imide group, and preferably includes a divalent aliphatic hydrocarbon group and a divalent aromatic hydrocarbon group.

In the group formed by R³²⁰ and R³³⁰ bonding to each other, examples of the divalent aliphatic hydrocarbon group include an alkylene group such as a methylene group, an ethylene group, a propylene group, and a trimethylene group.

In the group formed by R³²⁰ and R³³⁰ bonding to each other, examples of the divalent aromatic hydrocarbon group include an arylene group such as a phenylene group and a naphthylene group.

Regardless of whether or not R³²⁰ and R³¹⁰ are bonded to each other to form a ring structure, it is preferable that R³²⁰ and R³³⁰ have a carbon atom at a position where they bond to a nitrogen atom of the trisubstituted urea structure. The carbon atoms of R³²⁰ and R³³⁰ at the position where they bond to a nitrogen atom of the trisubstituted urea structure are preferably primary carbon atoms, secondary carbon atoms, tertiary carbon atoms, carbon atoms having aromaticity, or carbonyl carbon atoms.

An aromatic group contained in at least one of R³²⁰ and R³³⁰ preferably has a carbon atom having aromaticity at a position where it bonds to a nitrogen atom of the trisubstituted urea structure. The carbon atom having aromaticity is, for example, a carbon atom contained in a benzene ring. Examples of an aromatic group having a carbon atom with aromaticity at a position where it bonds to the outside include an aryl group such as a phenyl group, and an arylene group such as a phenylene group.

Examples of preferred trisubstituted urea compounds (III) include the following (III-I), (III-2), and (III-3).

In (III-1), in the above general formula (III), one or less of R³²⁰ and R³³⁰ is a monovalent organic group having no aromatic group, and at least one is a substituted or unsubstituted phenyl group. One of R³²⁰ and R³³⁰ may be a substituted or unsubstituted phenyl group, and the other may be a monovalent aliphatic hydrocarbon group. R³²⁰ and R³³⁰ may each independently be a substituted or unsubstituted phenyl group.

In (III-I), examples of the monovalent organic group having no aromatic group that is one or less of R³²⁰ and R³³⁰ include a substituted or unsubstituted monovalent aliphatic hydrocarbon group having 1 to 12 carbon atoms. Specific examples of the monovalent organic group having no aromatic group include a methyl group, an ethyl group, an n-propyl group, an isopropyl group, an n-butyl group, an isobutyl group, a sec-butyl group, a tert-butyl group, each isomer of a pentyl group, each isomer of a hexyl group, each isomer of a heptyl group, each isomer of an octyl group, each isomer of a nonyl group, each isomer of a decyl group, a cyclopentyl group, and a cyclohexyl group. Among these, an unsubstituted aliphatic hydrocarbon group having 1 to 12 carbon atoms is preferred, and a methyl group, an ethyl group, an n-propyl group, an isopropyl group, an n-butyl group, or an isobutyl group is preferred.

In (III-1), the number of substituents on the phenyl group represented by at least one of R³²⁰ and R³³⁰ is an integer from 0 to 5, more preferably 0 or 1, and still more preferably 0.

When at least one of R³²⁰ and R³³⁰ is a substituted phenyl group, examples of the substituent (hereinafter sometimes referred to as a "benzene ring substituent") include an aliphatic hydrocarbon group having 1 to 12 carbon atoms or an aromatic hydrocarbon group having 6 to 12 carbon atoms, which may be substituted or unsubstituted. The benzene ring substituent may have an ether group, a carbonyl group, or an ester group.

When the benzene ring substituent is an aliphatic hydrocarbon group or an aromatic hydrocarbon group, examples thereof include an alkyl group having 1 to 12 carbon atoms, an aryl group having 6 to 12 carbon atoms, or an aralkyl group having 7 to 12 carbon atoms.

When the benzene ring substituent has an ether group, examples thereof include an alkoxy group having 1 to 12 carbon atoms, an aryloxy group having 6 to 12 carbon atoms, or an aralkyloxy group having 7 to 12 carbon atoms.

When the benzene ring substituent has a carbonyl group, examples thereof include an alkylcarbonyl group having 1 to 12 carbon atoms, an arylcarbonyl group having 6 to 12 carbon atoms, or an aralkylcarbonyl group having 7 to 12 carbon atoms.

When the benzene ring substituent has an ester group, examples thereof include an alkoxycarbonyl group or alkylcarbonyloxy group having 1 to 12 carbon atoms, an aryloxycarbonyl group or arylcarbonyloxy group having 6 to 12 carbon atoms, or an aralkyloxycarbonyl group or aralkylcarbonyloxy group having 7 to 12 carbon atoms.

Specific examples the benzene ring substituent include a methyl group, an ethyl group, an n-propyl group, an isopropyl group, an n-butyl group, an isobutyl group, a sec-butyl group, a tert-butyl group, each isomer of a pentyl group, each isomer of a hexyl group, each isomer of a heptyl group, each isomer of an octyl group, each isomer of a nonyl group, each isomer of a decyl group, a cyclopentyl group, a cyclohexyl group, a phenyl group, a benzyl group, an o-tolyl group, an m-tolyl group, and a p-tolyl group. Among these, an unsubstituted aliphatic hydrocarbon group having 1 to 12 carbon atoms is preferred, and a methyl group, an ethyl group, an n-propyl group, an isopropyl group, an n-butyl group, or an isobutyl group is preferred.

(III-2) is a divalent organic group represented by a general formula -R³²¹-R³²²-in which R³²⁰ and R³³⁰ in the above general formula (III) are bonded to each other to form a ring structure via a carbon-carbon bond. One or less of R³²¹ and R³²² is a substituted or unsubstituted divalent aliphatic hydrocarbon group having 1 to 12 carbon atoms, and at least one of R³²¹ and R³²² is a substituted or unsubstituted o-phenylene group. One of R³²¹ and R³²² may be a substituted or unsubstituted o-phenylene group, and the other may be a divalent aliphatic hydrocarbon group. R³²¹ and R³²² may each independently be a substituted or unsubstituted o-phenylene group.

Specific examples of the divalent aliphatic hydrocarbon group represented by one or less of R³²¹ and R³²² include a methylene group, an ethylene group, a propylene group, and a trimethylene group.

The number of substituents on the o-phenylene group represented by at least one of R³²¹ and R³²² is an integer from 0 to 4, more preferably 0 or 1, and still more preferably 0. Examples of the substituent on the o-phenylene group represented by at least one of R³²¹ and R³²² include the same as those exemplified as the benzene ring substituents in (III-1).

(III-3) is a divalent organic group represented by a general formula -R³³¹-Z³³¹-R³³²- in which R¹²⁰ and R³³⁰ in the above general formula (III) are bonded to each other to form a ring structure via a carbon-carbon bond, a carbon-oxygen-carbon bond, or a carbon-nitrogen-carbon bond. Z³³¹ is -O-, -NH-, or -C(=O)-. One or less of R³³¹ and R³³² is a substituted or unsubstituted divalent aliphatic hydrocarbon group having 1 to 12 carbon atoms, and at least one of R³³¹ and R³³² is a substituted or unsubstituted o-phenylene group. One of R³³¹ and R³³² may be a substituted or unsubstituted o-phenylene group, and the other may be a divalent aliphatic hydrocarbon group. R³³¹ and R³³² may each independently be a substituted or unsubstituted o-phenylene group.

Specific examples of the divalent aliphatic hydrocarbon group represented by one or less of R³³¹ and R³³² include a methylene group, an ethylene group, a propylene group, and a trimethylene group.

The number of substituents on the o-phenylene group represented by at least one of R³³¹ and R³³² is an integer from 0 to 4, more preferably 0 or 1, and still more preferably 0. Examples of the substituent on the o-phenylene group represented by at least one of R³³¹ and R³³² include the same as those exemplified as the benzene ring substituents in (III-1).

Among the above general formulas (III), (III-1), (III-2), and (III-3), it is preferable that R³²⁰ and R³³⁰, as well as R³²¹, R³²², R³³¹, and R³³² do not contain an olefinic or acetylenic unsaturated carbon-carbon bond, as the effects of reducing coloration are further enhanced. The hydrocarbon groups contained in R³²⁰ and R³³⁰, as well as R³²¹, R³²², R³³¹, and R³³², are preferably saturated aliphatic hydrocarbon groups or aromatic hydrocarbon groups.

Further, from the viewpoint of thermal stability, it is preferable that R³²⁰ and R³³⁰, as well as R³²¹, R³²², R³³¹, and R³³², have a structure constituted solely of carbon atoms and hydrogen atoms, excluding Z³³¹ in (III-3).

In (III-1), it is preferable that one or less of R³²⁰ and R³³⁰ is a monovalent aliphatic hydrocarbon group, at least one of R¹²⁰ and R³³⁰ is an unsubstituted or monosubstituted phenyl group, and the substituent on the phenyl group is an unsubstituted aliphatic hydrocarbon group having 1 to 12 carbon atoms or aromatic hydrocarbon group having 6 to 12 carbon atoms.

In a more preferred form of (III-1), it is preferable that one or less of R³²⁰ and R³³⁰ is a monovalent aliphatic hydrocarbon group having 1 to 12 carbon atoms, at least one of R³²⁰ and R³³⁰ is an unsubstituted or monosubstituted phenyl group, and the substituent on the phenyl group is a methyl group or an ethyl group.

In (III-2), it is preferable that one or less of R³²¹ and R³²² is a divalent aliphatic hydrocarbon group, at least one of R³²¹ and R³²² is an unsubstituted or monosubstituted o-phenylene group, and the substituent on the o-phenylene group is an unsubstituted aliphatic hydrocarbon group having 1 to 12 carbon atoms or aromatic hydrocarbon group having 6 to 12 carbon atoms.

In a more preferred form of (III-2), it is preferable that one or less of R³²¹ and R³²² is a divalent aliphatic hydrocarbon group having 1 to 12 carbon atoms, at least one of R³²¹ and R³²² is an unsubstituted or monosubstituted o-phenylene group, and the substituent on the o-phenylene group is a methyl group or an ethyl group.

In (III-3), it is preferable that one or less of R³³¹ and R³³² is a divalent aliphatic hydrocarbon group, at least one of R³³¹ and R³³² is an unsubstituted or monosubstituted o-phenylene group, and the substituent on the o-phenylene group is an unsubstituted aliphatic hydrocarbon group having 1 to 12 carbon atoms or aromatic hydrocarbon group having 6 to 12 carbon atoms.

In a more preferred form of (III-3), it is preferable that one or less of R³³¹ and R³³² is a divalent aliphatic hydrocarbon group having 1 to 12 carbon atoms, at least one of R³³¹ and R³³² is an unsubstituted or monosubstituted o-phenylene group, and the substituent on the o-phenylene group is a methyl group or an ethyl group.

### [Method for producing trisubstituted urea compound (III)]

The trisubstituted urea compound (III) can be synthesized using a known production method. Examples of a method for synthesizing the trisubstituted urea compound (III) include a method in which an isocyanate group blocked with a secondary amine compound is generated during a production process of a blocked isocyanate compound.

Examples of the secondary amine compound used in the synthesis of the trisubstituted urea compound (III) include a compound represented by the following general formula (B).

H-N(-R³⁵³)-R³⁵² (B)

In the above general formula (B), R³⁵² and R³⁵³ are the same as those listed as R³²⁰ and R³³⁰ in the above general formula (III), respectively.

The trisubstituted urea compound (III) has active hydrogen and provides excellent storage stability and coloration inhibition for the polyisocyanate composition. The trisubstituted urea compound (III) inhibits the formation of a 1-nylon structure, which is the aforementioned "compound having UV absorption in a region of a decamer or higher isocyanate in GPC," and acts as a stabilizer during storage.

Because the trisubstituted urea compound (III) has an aromatic group in at least one of R³²⁰ and R³³⁰, the surrounding of the active hydrogen contained in the trisubstituted urea structure is bulky, and an addition reaction with an isocyanate group does not occur. Further, because the aromatic group has electron withdrawing properties, an anion generated when the active hydrogen contained in the trisubstituted urea structure is abstracted by a base is stabilized, making it possible to inhibit the formation of a 1-nylon structure.

Although it is preferable to increase the content of the trisubstituted urea compound (III) in order to improve the storage stability and coloration inhibition of the polyisocyanate composition 3, when the content of the trisubstituted urea compound (III) is too high, the production cost, applications, and the like of the polyisocyanate composition may be adversely affected.

The polyisocyanate composition 3 preferably contains 1.0 ppm by mass or more and 1.0 × 10⁴ ppm by mass or less of trisubstituted urea compound (III), based on the total mass of the polyisocyanate compounds.

The polyisocyanate composition 3 of the present embodiment can contain an optional component other than the trisubstituted urea compound (III) and the polyisocyanate compound, depending on the application, purpose, and the like.

The polyisocyanate composition 3 of the present embodiment can be used as a material for synthesizing polyurethane, a curing agent, and the like.

The polyisocyanate composition of the present embodiment can be utilized in a wide range of fields, including flexible foams, rigid foams, elastomers, adhesives, coating materials, and binders.

### <<Polyisocyanate composition 4>>

A polyisocyanate composition 4 of the present embodiment contains two or more compounds among the compounds represented by the general formulas (I), (II), and (III), and a polyisocyanate compound. Forms of the compounds represented by the general formulas (I), (II), and (III) are the same as those of the polyisocyanate compositions 1 to 3 described above.

The polyisocyanate composition 4 is more preferable because it is possible to provide a polyisocyanate with superior storage stability and coloration inhibition, as compared with the polyisocyanate compositions 1 to 3 described above. In particular, it is even more preferable when the polyisocyanate composition 4 contains compounds represented by the general formulas (I), (II), and (III) and a polyisocyanate compound, because a polyisocyanate with even superior storage stability and coloration inhibition can be provided.

In the polyisocyanate composition 4, the content of compound (I), compound (II), or compound (III), respectively, based on the total mass of the polyisocyanate compounds is preferably 1.0 ppm by mass or more and 5.0 × 10⁴ ppm by mass or less, and more preferably 1.0 ppm by mass or more and 1.0 × 10⁴ ppm by mass or less. Further, when the polyisocyanate composition 4 contains all of the compounds represented by the general formulas (I), (II), and (III), the total amount of the compounds represented by the general formulas (I), (II), and (III) based on the total mass of the polyisocyanate compounds is preferably 1.0 ppm by mass or more and 15.0 × 10⁴ ppm by mass or less, and more preferably 1.0 ppm by mass or more and 3.0 × 10⁴ ppm by mass or less.

### [Polyisocyanate compound]

The polyisocyanate compounds contained in the polyisocyanate compositions 1 to 4 are not particularly limited as long as they are compounds different from the compounds represented by the above general formulas (I), (II), and (III), but any compound having two or more isocyanate groups (-NCO) will suffice. Examples of the polyisocyanate compounds include compounds represented by the following general formula (P) (hereinafter sometimes referred to as a "polyisocyanate compound (P)").

R⁷⁰-(-NCO)ₚ (P)

In the above general formula (P), R⁷⁰ is a p-valent organic group that does not contain an isocyanate group, and p is an integer from 2 to 12.

R⁷⁰ is preferably a substituted or unsubstituted p-valent aliphatic hydrocarbon group having 1 to 70 carbon atoms or p-valent aromatic hydrocarbon group having 6 to 70 carbon atoms, which may have an ether group, a carbonyl group, an ester group, an imino group (-NH-), an amide group, or an imide group. p is preferably 2 or 3.

When an aliphatic hydrocarbon group is selected for R⁷⁰, the number of carbon atoms is preferably from 1 to 70, more preferably from 1 to 20, still more preferably from 1 to 12, and particularly preferably from 1 to 10.

When an aromatic hydrocarbon group is selected for R⁷⁰, the number of carbon atoms is preferably from 6 to 70, more preferably from 6 to 20, still more preferably from 6 to 12, and particularly preferably from 6 to 10.

The polyisocyanate compound (P) may be a compound that does not have a blocked isocyanate group. In this case, R⁷⁰ is an organic group that does not contain a blocked isocyanate group.

The blocked isocyanate group is a group that can thermally dissociate into an isocyanate group and a blocking agent represented by the general formula BL-H. The blocking agent is a compound having active hydrogen. Examples of the blocking agent include a phenol-based blocking agent, an alcohol-based blocking agent, a thiol-based blocking agent, an amine-based blocking agent, an ammonia-based blocking agent, an oxime-based blocking agent, a hydroxylamine-based blocking agent, and an active methylene-based blocking agent.

The polyisocyanate compound (P) may or may not contain a unit constituting an isocyanate polymer in R⁷⁰. Among units containing a nitrogen atom derived from one or more isocyanate groups, the unit constituting an isocyanate polymer refers to an isocyanurate group, a biuret group, an iminooxadiazinedione group, a ureylene group, an allophanate group, a uretdione group, and a urethane group.

The polyisocyanate compound (P) may be a compound represented by the following general formula (P-a).

(OCN-)ₚ₁-R⁷¹-L⁷¹-R⁷²-(-NCO)ₚ₂ (P-a)

In the above general formula (P-a), R⁷¹ is a (p1 + 1)-valent cyclic aliphatic hydrocarbon group or aromatic hydrocarbon group, L⁷¹ is a single bond or a divalent acyclic aliphatic hydrocarbon group, and R⁷² is a (p2 + 1)-valent cyclic aliphatic hydrocarbon group or aromatic hydrocarbon group. Within a range in which p1 + p2 is an integer of 2 or more and 12 or less, p1 and p2 are each an integer of 1 or more.

The polyisocyanate compound (P) may be a compound represented by the following general formula (P-b).

(OCN-)ₚ₃-R⁷³-C³ -R⁷⁴-(-NCO)ₚ₄ (P-b)

In the above general formula (P-b), R⁷³ is a (p3 + 1)-valent aliphatic hydrocarbon group or aromatic hydrocarbon group, L⁷³ is an ether group, a carbonyl group, an ester group, an imino group (-NH-), an amide group, or an imide group, and R⁷⁴ is a (p4 + 1)-valent aliphatic hydrocarbon group or aromatic hydrocarbon group. Within a range in which p3 + p4 is an integer of 2 or more and 12 or less, p3 and p4 are each an integer of 0 or more.

Examples of the aliphatic hydrocarbon group or aromatic hydrocarbon group represented by R⁷¹, R⁷², R⁷³, R⁷⁴, and L⁷¹ include the same groups as those exemplified as the aliphatic hydrocarbon group or aromatic hydrocarbon group represented by R⁷⁰.

Specific examples of the polyisocyanate compound include pentamethylene diisocyanate (hereinafter sometimes referred to as "PDI"), hexamethylene diisocyanate (hereinafter sometimes referred to as "HDI"), isophorone diisocyanate (hereinafter sometimes referred to as "IPDI"), 4,4'-methylenebis(cyclohexane isocyanate) (hereinafter sometimes referred to as "MBCI"), diisocyanatotoluene (hereinafter sometimes referred to as "TDI"), 4,4'-diphenylmethane diisocyanate (hereinafter sometimes referred to as "MDI"), 4-isocyanatomethyl-1,8-octamethylene diisocyanate (hereinafter sometimes referred to as "TTI"), lysine triisocyanate (hereinafter sometimes referred to as "LTI"), and lysine diisocyanate (hereinafter sometimes referred to as "LDI").

### <Method for producing isocyanate compound>

A method for producing an isocyanate compound of the present embodiment includes a reaction step of decomposing a blocked isocyanate compound into a blocking agent and an isocyanate compound by heat treatment in the presence of a compound having a structure represented by either one or both of the following general formulas (I) and (II), thereby obtaining the isocyanate compound. 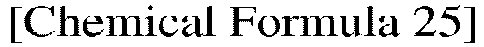 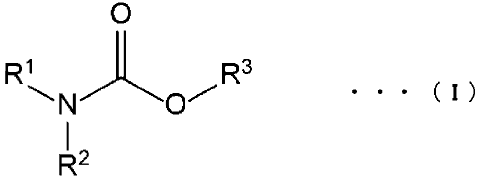 (In the general formula (I), R¹ and R² each independently represent a monovalent organic group; R¹ and R² may each independently form a ring structure via a carbon-carbon bond, a carbon-oxygen-carbon bond, or a carbon-nitrogen-carbon bond; and R³ represents an aliphatic hydrocarbon group or a hydrocarbon group having an aromatic group.) 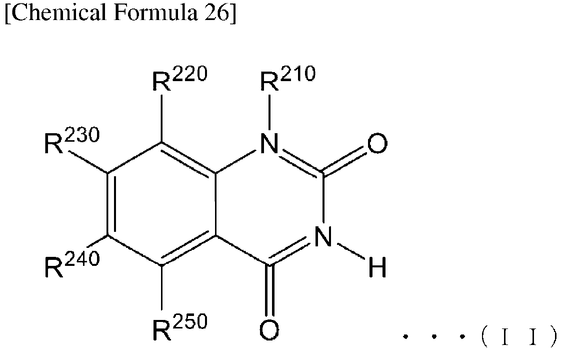 (In the general formula (II), R²¹⁰ is a monovalent organic group, and R²²⁰, R²³⁰, R²⁴⁰, and R²⁵⁰ each independently represent a monovalent organic group or hydrogen; and R²¹⁰ and R²²⁰ may be bonded to each other to form a ring structure via a carbon-carbon bond, a carbon-oxygen-carbon bond, or a carbon-nitrogen-carbon bond.)

### <<Isocyanate compound production method 1>>

The isocyanate compound production method 1 includes a reaction step of decomposing a blocked isocyanate compound into a blocking agent and an isocyanate compound by heat treatment in the presence of a compound having a structure represented by the above general formula (I), thereby obtaining the isocyanate compound.

The inventors of the present invention and others discovered that a high yield method for producing an isocyanate compound can be achieved without increasing the amount of by-products produced by decomposing a blocked isocyanate compound into a blocking agent and an isocyanate compound by heat treatment in the presence of the compound (I) to produce the isocyanate compound, thereby leading to completion of the present invention.

The production method of the present invention will be described below.

In the reaction step in the isocyanate compound production method according to the present embodiment, a blocked isocyanate compound is decomposed into a blocking agent and an isocyanate compound by heat treatment in the presence of the compound (I), thereby obtaining the isocyanate compound.

The compound (I) represented by the above-mentioned general formula (I) is used as the compound (I). In particular, when R³ in the general formula (I) is an aromatic hydrocarbon group, it is more preferable because the reactivity between the compound (I) and an organic amine produced by decomposition of the ureylene group is improved, thereby enabling efficient reduction of the ureylene group.

When an aromatic hydrocarbon group is selected as R³ for the compound (I) used in the isocyanate compound production method 1, the number of carbon atoms is preferably from 6 to 70, more preferably from 6 to 20, still more preferably from 6 to 12, and particularly preferably from 6 to 10.

Examples of the aromatic hydrocarbon group represented by R³ include an aryl group and aralkyl group, which may be unsubstituted or substituted. Examples of the substituent on these aromatic hydrocarbon groups include an aliphatic hydrocarbon group, a hydroxyl group, a cyano group, and a halogen atom. Examples of the aliphatic hydrocarbon group selected as the substituent on the aromatic hydrocarbon group represented by R³ include the same groups as those exemplified as the aliphatic hydrocarbon group represented by R³. Examples of the halogen atom as a substituent include a fluorine atom, a chlorine atom, a bromine atom, and an iodine atom.

Specific examples of the aromatic hydrocarbon group represented by R³ include a phenyl group, a naphthyl group, an anthryl group, a pyrenyl group, a phenanthryl group, an o-tolyl group, an m-tolyl group, a p-tolyl group, a benzyl group, and a phenethyl group.

Ureylene groups react with isocyanates, for example, according to the reactions represented by the following formulas (70), (71), and (72), thereby producing by-products.

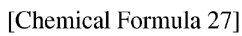

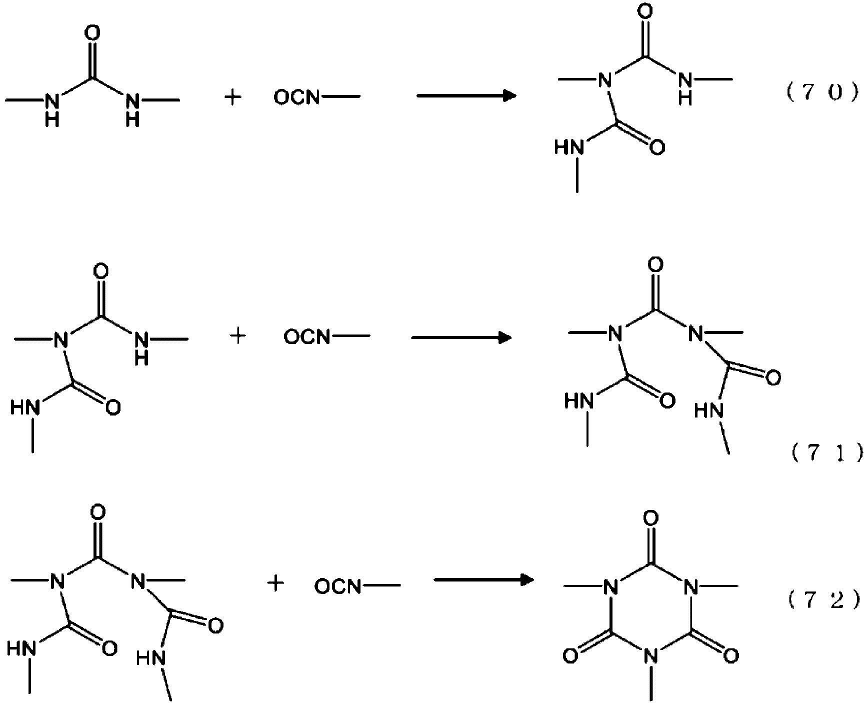

The compound (I) reacts with ureylene groups to inhibit the production of by-products caused by the ureylene groups. The reaction between the compound (I) and a ureylene group is represented by either one or both of the following general formula A and the following general formula B.

Here, it is presumed that the ureylene group decomposes into an amino group and an isocyanate group through thermal dissociation, and the produced amino group reacts with the compound (I), thereby allowing either one or both of the reactions represented by the following general formula A and the following general formula B to proceed. Whether the reaction represented by the general formula A or the general formula B predominates depends on the leaving ability of the group bonded to the carbonyl, and therefore the reaction represented by the general formula A predominates.

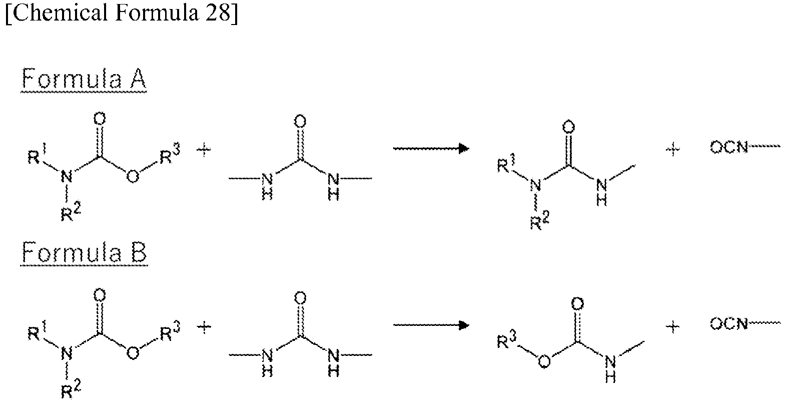

Here, when the reaction represented by the general formula B proceeds, the obtained carbamic acid ester reacts with an isocyanate through the reaction represented by the following general formula C, thereby allowing reactions such as those shown in, for example, (67), (68), and (69) to proceed.

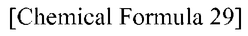

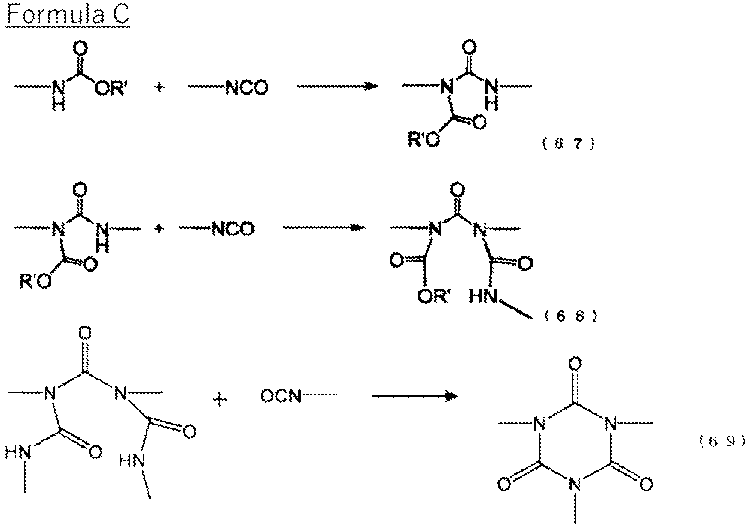

On the other hand, when the reaction represented by the general formula A proceeds, the obtained trisubstituted urea group reacts only slightly or not at all with the aforementioned isocyanate. While the reason for this is unclear, the reason is presumed to be significant steric hindrance of the obtained trisubstituted urea group.

In the reaction step, from the viewpoint of improving the thermal decomposition rate, the amount of the compound (I) present is preferably large. More specifically, the amount of the compound (I) present is preferably 1 ppm by mass or more, more preferably 100 ppm by mass or more, still more preferably 1,000 ppm by mass or more, and especially preferably 10,000 ppm by mass or more, with respect to the blocked isocyanate compound. Further, from the viewpoint of inhibiting the production of by-products due to ureylene groups, the amount of the compound (I) present is preferably 0.1 mol% or more, more preferably 1 mol% or more, still more preferably 10 mol% or more, and especially preferably 100 mol% or more, with respect to the ureylene groups.

The reaction temperature (thermal decomposition temperature of the blocked isocyanate compound) in the reaction step is not particularly limited and is appropriately selected depending on the rate at which the blocked isocyanate compound decomposes into the blocking agent and the isocyanate compound, and the degree of thermal denaturation and coloration. From the viewpoint of inhibiting denaturation of the isocyanate compound, the reaction temperature is preferably 350°C or lower, more preferably 300°C or lower, and still more preferably 260°C or lower. On the other hand, when the reaction temperature is low, since it is required to set the temperature of a condenser to a low temperature, which may require new equipment, from this viewpoint, a temperature of 50°C or higher is preferred, 80°C or higher is more preferred, and 100°C or higher is still more preferred.

The reaction pressure in the reaction step varies depending on the type of compound used and the reaction temperature, but may be any one of reduced pressure, normal pressure, and elevated pressure, and it is typically carried out within a range of an absolute pressure of 20 Pa or higher and 2 × 10⁷ Pa or lower.

The reaction step may be carried out in the presence of oxygen. When the reaction step is carried out in the presence of oxygen, since thermal denaturation and discoloration of the isocyanate compound are caused, the amount of oxygen present in an apparatus for the thermal decomposition of blocked isocyanate compounds is preferably reduced. On the other hand, when considering large-scale production equipment, reduction of air leakage into the production equipment is required in order to reduce the amount of oxygen present in the apparatus for the thermal decomposition of blocked isocyanate compounds, and for this reason, equipment design criteria become stricter to cause an increase in the cost of equipment. From this viewpoint, it is preferable to control the oxygen concentration in the gas supplied to the reaction step under a high condition, preferably above 0% by volume, more preferably above 0.0001% by volume, and still more preferably above 0.001 % by volume.

In the reaction step, any solvent may be used in any proportion. As the solvent, an inert solvent having no reactivity with blocked isocyanate compounds and the like is preferred. Such a solvent is preferably an ester-based solvent, an ether-based solvent, a phosphoric acid ester-based solvent, a hydrocarbon-based solvent, an aromatic hydrocarbon-based solvent, or a carbonic acid derivative-based solvent.

In the reaction step, a reaction solution may contain any metal in any proportion. The form of the metal may be a complex or a solid. While the metal reduces the thermal decomposition temperature of the blocked isocyanate compound, it may also cause thermal denaturation, deterioration, and coloration. The content of the metal is preferably less than 10% by mass, more preferably less than 1% by mass, and still more preferably less than 0.1 ppm by mass, with respect to the mass of the blocked isocyanate compound.

In the reaction step, the reaction solution may contain an organic acid, an inorganic acid, an organic base, or an inorganic base. These acids and bases act as catalysts in the thermal decomposition of the blocked isocyanate, making it possible to reduce the temperature required for thermal decomposition. On the other hand, these acids and bases also act as catalysts for side reactions in the thermal decomposition of the blocked isocyanate. From these viewpoints, the contents of these organic acids, inorganic acids, organic bases, and inorganic bases are preferably less than 10% by mass, more preferably less than 1% by mass, still more preferably less than 0.1 % by mass, and particularly preferably less than 1 ppb by mass, with respect to the mass of the blocked isocyanate compound.

The thermal decomposition apparatus for the blocked isocyanate compound is not particularly limited, and a known thermal decomposition apparatus can be used. For example, an apparatus in which a composition containing a blocked isocyanate compound is placed in a container connected to a condensing unit, the container is heated to thermally decompose the blocked isocyanate compound, and vapor containing the blocking agent produced after, or simultaneously with, the thermal decomposition is introduced into the condensing unit to separate the isocyanate compound and the blocking agent in a batchwise manner; an apparatus in which a composition containing a blocked isocyanate compound is introduced continuously into a distillation column heated to or above the thermal decomposition temperature of the blocked isocyanate compound, the blocking agent and isocyanate compound produced simultaneously with the thermal decomposition of the blocked isocyanate compound are separated to continuously obtain free isocyanate compound and the blocking agent; an apparatus in which a blocked isocyanate compound is introduced into an evaporator or thin film heated to or above the thermal decomposition temperature of the blocked isocyanate compound, vapor containing the blocking agent and isocyanate compound produced by thermal decomposition is introduced into a distillation column to separate the blocking agent and isocyanate compound in the distillation column; and the like can be used.

In a thermal decomposition apparatus for blocked isocyanate compounds, a material for a portion that comes into contact with the composition containing the blocked isocyanate compound and the blocking agent, isocyanate compound, and other components produced during thermal decomposition may be any known material, as long as it does not adversely affect the denaturation and the like of the blocked isocyanate compound, blocking agent, isocyanate compound, and other components. Specific examples of the material include steel, stainless steel, ceramic, carbon, and a material lined with these materials.

### [Blocked isocyanate compound]

A blocked isocyanate compound is a compound that can dissociate into a blocking agent and an isocyanate compound by heat, as shown in the following reaction formula.

R^{a}-(NH-C(=O)-BL)ₙₐ → BL-H + R^{a}-(N=C=O)ₙₐ

In the above reaction formula, BL-H is a blocking agent having active hydrogen. R^{a} is an na-valent organic group, and na is an integer of 1 or more. BL- is a residue obtained by removing the active hydrogen from the blocking agent.

### [Isocyanate compound]

As the isocyanate compound in the reaction step, an isocyanate compound represented by the following general formula (IV) (hereinafter sometimes referred to as an "isocyanate compound (IV)") is preferably used. 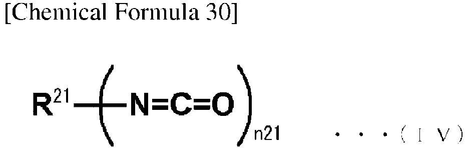 (In the general formula (IV), R²¹ is an n21-valent organic group; and n21 is an integer from 1 to 12.)

R²¹ is not particularly limited as long as it is an organic group with a valence from 1 to 12, but is preferably an organic group (hydrocarbon group) composed of a carbon atom and a hydrogen atom, or an organic group composed of a carbon atom, an oxygen atom, and a hydrogen atom, and more preferably an organic group without active hydrogen. The oxygen atom included in R²¹ preferably constitutes an ether group or an ester group.

The aliphatic hydrocarbon group represented by R²¹ is preferably an alkyl group, an alkylene group, an alkanetriyl group, a cycloalkyl group, a cycloalkylene group, a cycloalkanetriyl group, or a group composed of two or more of these groups.

The aromatic hydrocarbon group represented by R²¹ is preferably a substituted or unsubstituted group having an aromatic ring with 6 to 13 carbon atoms. Examples of the substituent include an alkyl group, an aralkyl group, an aryl group, an alkoxy group, an alkoxycarbonyl group, and an alkylcarbonyloxy group.

Examples of the isocyanate compound (IV) include a monofunctional isocyanate compound, a bifunctional isocyanate compound, and a polyfunctional isocyanate compound.

Examples of the monovalent organic group represented by R²¹ in a monofunctional isocyanate compound include a substituted or unsubstituted alkyl group, cycloalkyl group, aralkyl group, and aryl group.

Examples of the divalent organic group represented by R²¹ in a bifunctional isocyanate compound include a substituted or unsubstituted alkylene group, cycloalkylene group, arylene group, arylenedialkylene group, alkylenediarylene group, and alkylenedicycloalkylene group. The bifunctional isocyanate compound may be a compound having an isocyanatoalkyl group such as isophorone diisocyanate, a compound having an isocyanatocycloalkyl group such as dicyclohexylmethane 4,4'-diisocyanate, a compound having an isocyanatoaryl group such as diphenylmethane diisocyanate, or a compound having a carbonyl group such as lysine diisocyanate.

Examples of the polyvalent organic group represented by R²¹ in a polyfunctional isocyanate compound include a substituted or unsubstituted alkanetriyl group, cycloalkanetriyl group, and arenetriyl group. The polyfunctional isocyanate compound may be a compound having an isocyanatoalkyl group such as 4-isocyanatomethyl-1,8-octamethylene diisocyanate, or a compound having a carbonyl group such as lysine triisocyanate.

### [Blocking agent]

The blocking agent is a compound having active hydrogen. The blocking agent in the reaction step preferably contains one or more compounds selected from the group consisting of a hydroxy compound, an amine compound, and ammonia. Further, examples of the hydroxy compound include one or more compounds selected from the group consisting of an aromatic hydroxy compound and an aliphatic hydroxy compound.

### (Blocking agent as aromatic hydroxy compound)

Examples of the aromatic hydroxy compound preferred as a blocking agent include an aromatic hydroxy compound represented by the following general formula (V). 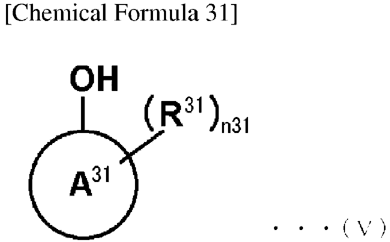 (In the general formula (V), a ring A³¹ represents an aromatic hydrocarbon ring having 6 to 20 carbon atoms; R¹¹ represents a hydrogen atom, a halogen atom, a carboxy group, an alkyl group having 1 to 20 carbon atoms, an alkoxy group having 1 to 20 carbon atoms, an alkyloxycarbonyl group having 1 to 20 carbon atoms, an alkylcarbonyloxy group having 1 to 20 carbon atoms, an aryl group having 6 to 20 carbon atoms, an aryloxy group having 6 to 20 carbon atoms, an aralkyl group having 7 to 20 carbon atoms, or an aralkyloxy group having 7 to 20 carbon atoms; R¹¹ may be bonded with the ring A³¹ to form a ring structure; and n31 is an integer from 1 to 10.)

The ring A³¹ may be a monocyclic ring or a polycyclic ring such as a condensed ring. Specific examples of the ring A³¹ include a benzene ring, a naphthalene ring, an anthracene ring, a phenanthrene ring, a naphthacene ring, a chrysene ring, a pyrene ring, a triphenylene ring, a pentalene ring, an azulene ring, a heptalene ring, an indacene ring, a biphenylene ring, an acenaphthylene ring, an aceanthrylene ring, and an acephenanthrylene ring. Of these, the ring A³¹ is preferably a benzene ring, a naphthalene ring, or an anthracene ring, and more preferably a benzene ring.

The hydroxy group shown in the general formula (V) is bonded to a carbon atom having aromaticity in the ring A³¹, thereby exhibiting phenolic properties. The aromatic hydrocarbon group having the ring A³¹ and n31 R³¹ groups may be a substituted or unsubstituted monovalent aromatic hydrocarbon group such as an aryl group.

R³¹ is a substituent of the ring A³¹, excluding the case of a hydrogen atom. As shown in the general formula (V), the ring A³¹ has one hydroxy group and n31 R³¹ groups. The n31 R³¹ groups may each be independently selected, different from each other, from the groups exemplified for R¹¹, or two or more of the same group may be selected therefrom. In addition to the above R³¹, the ring A³¹ may also have a hydrogen atom and/or substituent bonded to a carbon atom constituting the ring A³¹. The hydrogen atom, substituent, and functional group bonded to a carbon atom constituting the ring A³¹ may be only one hydroxy group and n31 R³¹ groups as shown in the general formula (V).

### (Blocking agent as aliphatic hydroxy compound)

Examples of the aliphatic hydroxy compound preferred as a blocking agent include an aliphatic hydroxy compound represented by the following general formula (VI).
[Chemical Formula 32]

R⁴¹-OH· (V1)

(In the general formula (VI), R⁴¹ represents a substituted or unsubstituted aliphatic hydrocarbon group having 1 to 24 carbon atoms, which may have an ether group, a carbonyl group, or an ester group.)

R⁴¹ is a monovalent aliphatic hydrocarbon group. The number of carbon atoms in the aliphatic hydrocarbon group represented by R⁴¹ is from 1 to 24, preferably from 1 to 20, and more preferably from 1 to 12. The aliphatic hydrocarbon group represented by R⁴¹ may be saturated or unsaturated. One hydroxy group shown in the general formula (VI) is bonded to a saturated carbon atom in R⁴¹, thereby exhibiting alcoholic properties.

### (Blocking agent as secondary amine compound)

Examples of the secondary amine compound preferred as a blocking agent include a secondary amine compound represented by the following general formula (VII). 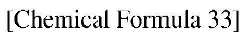 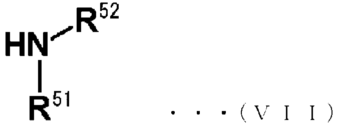 (In the general formula (VII), R⁵¹ and R⁵² each independently represent a monovalent organic group; R⁵¹ and R⁵² may be bonded to each other to form a ring structure via a carbon-carbon bond, a carbon-oxygen-carbon bond, or a carbon-nitrogen-carbon bond.)

In particular, R⁵¹ and R⁵² are preferably substituted or unsubstituted monovalent aliphatic hydrocarbon groups having 1 to 70 carbon atoms or monovalent aromatic hydrocarbon groups having 6 to 70 carbon atoms, which may have an ether group, a carbonyl group, or an ester group. R⁵¹ and R⁵² are preferably organic groups that do not contain active hydrogen, and more preferably substituted or unsubstituted organic groups that do not contain an amino group.

Examples of the aliphatic hydrocarbon group represented by R⁵¹ and R⁵² include an alkyl group and a cycloalkyl group. The number of carbon atoms in the aliphatic hydrocarbon group represented by R⁵¹ and R⁵² is preferably from 1 to 70, more preferably from 1 to 20, still more preferably from 1 to 12, and particularly preferably from 1 to 10. Examples of the substituent on the aliphatic hydrocarbon group represented by R⁵¹ and R⁵² include a hydroxyl group, a cyano group, and a halogen atom.

Examples of the aromatic hydrocarbon group represented by R⁵¹ and R⁵² include an aryl group and an aralkyl group. The number of carbon atoms in the aromatic hydrocarbon group represented by R⁵¹ and R⁵² is preferably from 6 to 70, more preferably from 6 to 20, still more preferably from 6 to 12, and particularly preferably from 6 to 10. Examples of the substituent on the aromatic hydrocarbon group represented by R⁵¹ and R⁵² include an aliphatic hydrocarbon group, a hydroxyl group, a cyano group, and a halogen atom.

When R⁵¹ and R⁵² are bonded to each other to form a ring structure, a group formed by R⁵¹ and R⁵² bonding to each other is a divalent organic group. Examples of the group formed by R⁵¹ and R⁵² bonding to each other include a substituted or unsubstituted divalent aliphatic hydrocarbon group having 1 to 70 carbon atoms or divalent aromatic hydrocarbon group having 6 to 70 carbon atoms, which may have an ether group, a carbonyl group, an ester group, a substituted imino group (-N(-R⁵³)-), a - CH=N- group, a substituted amide group (-C(=O)-N(-R⁵⁴)-), or a substituted imide group (-C(=O)-N(-R⁵⁵)-C(=O)-). Examples of R^{53,} R⁵⁴, and R⁵⁵ include a monovalent aliphatic hydrocarbon group or an aromatic hydrocarbon group.

### <<Isocyanate compound production method 2>>

A method for producing an isocyanate compound according to the present embodiment includes a reaction step of decomposing a blocked isocyanate compound into a blocking agent and an isocyanate compound by heat treatment in the presence of a compound having a structure represented by the above general formula (II) (hereinafter sometimes referred to as a "quinazolinedione structure (II)"), thereby obtaining the isocyanate compound.

The inventors of the present invention and others discovered that the problem of thermal decomposition rate can be solved, without increasing the amount of by-products produced, by producing an isocyanate compound by decomposing a blocked isocyanate compound into a blocking agent and an isocyanate compound by heat treatment in the presence of a compound having a quinazolinedione structure (II), thereby leading to completion of the present invention.

### [Reaction step]

In the reaction step in the method for producing an isocyanate compound according to the present embodiment, a blocked isocyanate compound is decomposed into a blocking agent and an isocyanate compound by heat treatment in the presence of a compound having a quinazolinedione structure (II), thereby obtaining the isocyanate compound.

In the reaction step, from the viewpoint of improving the thermal decomposition rate, the amount of the compound having a quinazolinedione structure (II) present is preferably large. More specifically, the amount of the compound having a quinazolinedione structure (II) present is preferably 1 ppm by mass or more, more preferably 100 ppm by mass or more, still more preferably 1,000 ppm by mass or more, and especially preferably 10,000 ppm by mass or more, with respect to the blocked isocyanate compound.

The explanations regarding the blocked isocyanate compound, isocyanate compound, and blocking agent in the isocyanate compound production method 2 are the same as those regarding the blocked isocyanate compound, isocyanate compound, and blocking agent in the above isocyanate compound production method 1.

The reaction temperature (thermal decomposition temperature of the blocked isocyanate compound) in the reaction step is not particularly limited and is appropriately selected depending on the rate at which the blocked isocyanate compound decomposes into the blocking agent and the isocyanate compound, and the degree of thermal denaturation and coloration. From the viewpoint of inhibiting denaturation of the isocyanate compound, the reaction temperature is preferably 350°C or lower, more preferably 300°C or lower, and still more preferably 260°C or lower. On the other hand, when the reaction temperature is low, since it is required to set the temperature of a condenser to a low temperature, which may require new equipment, from this viewpoint, a temperature of 50°C or higher is preferred, 80°C or higher is more preferred, and 100°C or higher is still more preferred.

The reaction pressure in the reaction step varies depending on the type of compound used and the reaction temperature, but may be any one of reduced pressure, normal pressure, and elevated pressure, and it is typically carried out within a range of an absolute pressure of 20 Pa or higher and 2 × 10⁷ Pa or lower.

In the reaction step, any solvent may be used in any proportion. As the solvent, an inert solvent having no reactivity with blocked isocyanate compounds and the like is preferred. Such a solvent is preferably an ester-based solvent, an ether-based solvent, a phosphoric acid ester-based solvent, a hydrocarbon-based solvent, an aromatic hydrocarbon-based solvent, or a carbonic acid derivative-based solvent.

In the reaction step, a reaction solution may contain any metal in any proportion. The form of the metal may be a complex or a solid. While the metal reduces the thermal decomposition temperature of the blocked isocyanate compound, it may also cause thermal denaturation, deterioration, and coloration. The content of the metal is preferably less than 10% by mass, more preferably less than 1% by mass, and still more preferably less than 0.1 ppm by mass, with respect to the mass of the blocked isocyanate compound.

In the reaction step, the reaction solution may contain an organic acid, an inorganic acid, an organic base, or an inorganic base. These acids and bases act as catalysts in the thermal decomposition of the blocked isocyanate, making it possible to reduce the temperature required for thermal decomposition. On the other hand, these acids and bases also act as catalysts for side reactions in the thermal decomposition of the blocked isocyanate. From these viewpoints, the contents of these organic acids, inorganic acids, organic bases, and inorganic bases are preferably less than 10% by mass, more preferably less than 1% by mass, still more preferably less than 0.1% by mass, and particularly preferably less than 1 ppb by mass, with respect to the mass of the blocked isocyanate compound.

The thermal decomposition apparatus for the blocked isocyanate compound is not particularly limited, and a known thermal decomposition apparatus can be used. For example, an apparatus in which a composition containing a blocked isocyanate compound is placed in a container connected to a condensing unit, the container is heated to thermally decompose the blocked isocyanate compound, and vapor containing the blocking agent produced after, or simultaneously with, the thermal decomposition is introduced into the condensing unit to separate the isocyanate compound and the blocking agent in a batchwise manner; an apparatus in which a composition containing a blocked isocyanate compound is introduced continuously into a distillation column heated to or above the thermal decomposition temperature of the blocked isocyanate compound, the blocking agent and isocyanate compound produced simultaneously with the thermal decomposition of the blocked isocyanate compound are separated to continuously obtain free isocyanate compound and the blocking agent; an apparatus in which a blocked isocyanate compound is introduced into an evaporator or thin film heated to or above the thermal decomposition temperature of the blocked isocyanate compound, vapor containing the blocking agent and isocyanate compound produced by thermal decomposition is introduced into a distillation column to separate the blocking agent and isocyanate compound in the distillation column; and the like can be used.

In a thermal decomposition apparatus for blocked isocyanate compounds, a material for a portion that comes into contact with the composition containing the blocked isocyanate compound and the blocking agent, isocyanate compound, and other components produced during thermal decomposition may be any known material, as long as it does not adversely affect the denaturation and the like of the blocked isocyanate compound, blocking agent, isocyanate compound, and other components. Specific examples of the material include steel, stainless steel, ceramic, carbon, and a material lined with these materials.

### <<Isocyanate compound production method 3>>

The isocyanate compound production method 3 of the present embodiment includes a reaction step in which a blocked isocyanate compound is decomposed into a blocking agent and an isocyanate compound by heat treatment in the presence of a compound having a structure represented by both of the above general formulas (I) and (II), thereby obtaining the isocyanate compound.

In the reaction step in which a blocked isocyanate compound is decomposed into a blocking agent and an isocyanate compound by heat treatment in the presence of a compound having a structure represented by both of the general formulas (I) and (II) to obtain an isocyanate compound, the amount of the compound (I) or (II) present is preferably 1 ppm by mass or more respectively, more preferably 100 ppm by mass or more respectively, still more preferably 1,000 ppm by mass or more respectively, and especially preferably 10,000 ppm by mass or more respectively, with respect to the blocked isocyanate compound. Further, from the viewpoint of inhibiting the production of by-products due to ureylene groups, the amount of the compound (I) present is preferably 0.1 mol% or more, more preferably 1 mol% or more, still more preferably 10 mol% or more, and especially preferably 100 mol% or more, with respect to the ureylene groups.

The explanations regarding the blocked isocyanate compound, isocyanate compound, and blocking agent in the isocyanate compound production method 3 are the same as those regarding the blocked isocyanate compound, isocyanate compound, and blocking agent in the above isocyanate compound production method 1.

### Examples

Hereinafter, the present invention will be described in more detail with reference to Examples, but the present invention is not limited to these Examples. It should be noted that purified products were used for all reagents used as raw materials.

### <Production of polyisocyanate composition 1>

A polyisocyanate compound, a compound (I), and a reaction terminator were mixed to produce polyisocyanate compositions of Examples 1 to 26 and Comparative Examples 1 to 2.

The amounts of each component mixed and specific materials are listed in Tables 1 to 2.

Any one of HDI, IPDI, PDI, MBCI, TDI, MDI, TTI, LTI, and LDI was used as the polyisocyanate compound.

Dibutyl phosphate (DBP) was used as the reaction terminator.

The following compounds (I)-1 to (I)-11 were used as the compound (I).

In the following chemical formulas, each symbol refers to the following group.
- Me: methyl group
- Et: ethyl group
- n-Bu: n-butyl group
- i-Pr: isopropyl group
- Oc: octyl group
- Cumyl: cumyl group

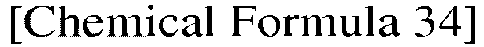

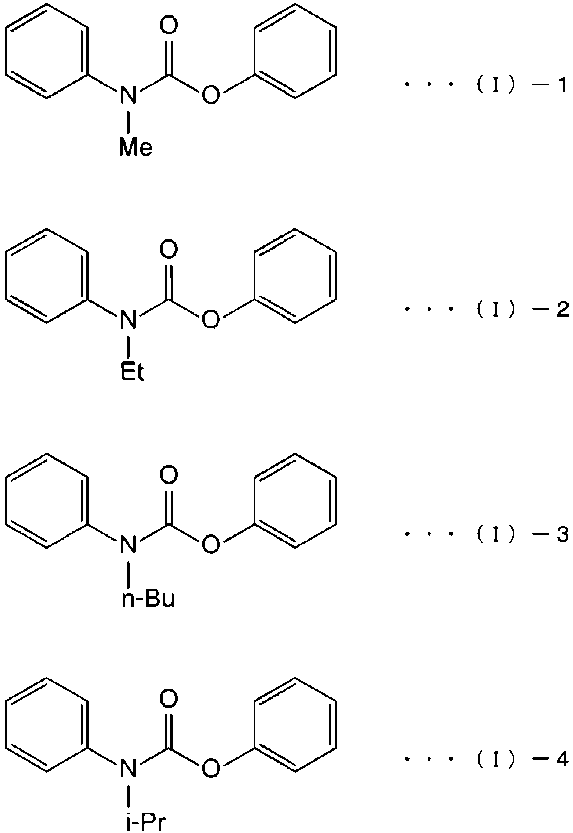

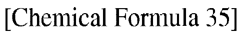

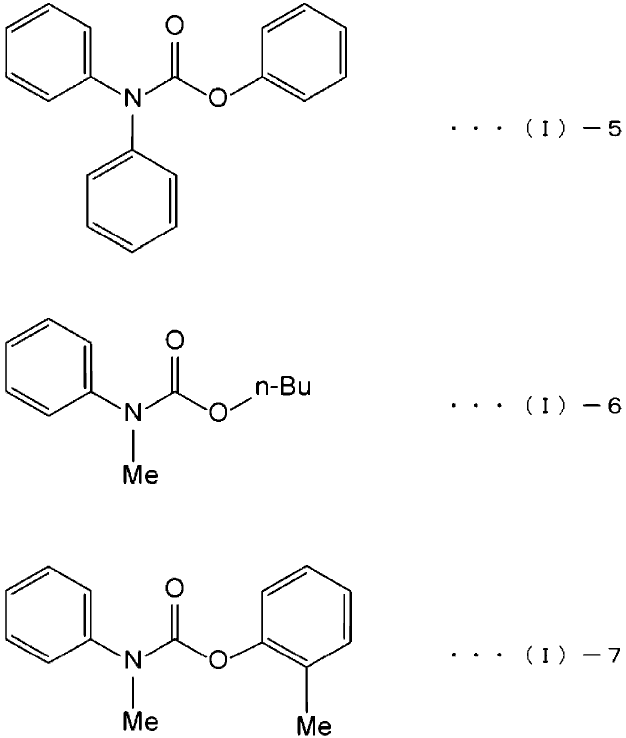

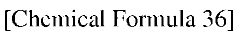

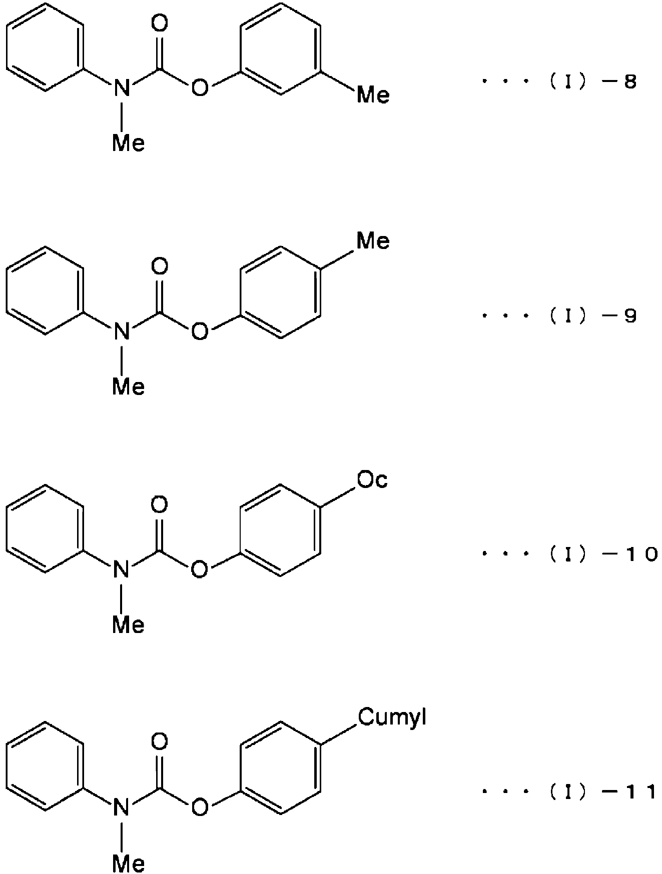

### <Evaluation of coloration inhibition>

The polyisocyanate compositions before and after storage were subjected to a color test using the Hazen unit color number (APHA rank) to evaluate coloration inhibition.

More specifically, the APHA rank before storage was measured using a sample prepared by dissolving 1 g of the obtained polyisocyanate composition in 2 g of benzyl toluene. Based on the values measured using a Hazen meter, ranking was carried out according to the following evaluation criteria.

### (Evaluation criteria)

Rank 1: APHA 0 or more, less than 5
Rank 2: APHA 5 or more, less than 10
Rank 3: APHA 10 or more, less than 15
Rank 4: APHA 15 or more, less than 20
Rank 5: APHA 20 or more, less than 25
Rank 6: APHA 25 or more, less than 30
Rank 7: APHA 30 or more, less than 35
Rank 8: APHA 35 or more, less than 40
Rank 9: APHA 40 or more, less than 45
Rank 10: APHA 45 or more, less than 50

The APHA rank after 300 days of storage was measured using a sample prepared by dissolving 1 g of the polyisocyanate composition after 300 days of storage described above in 2 g of benzyltoluene. Based on the values measured using a Hazen meter, ranking was carried out according to the same evaluation criteria as in Evaluation 1.

### <Evaluation of storage stability>

GPC measurement was performed using polyisocyanate compositions after completion of a 300-day storage period as samples, and storage stability was evaluated using the following area ratio.
Area ratio: (B)/(A)
(In the above area ratio, (A) is an area of the peak corresponding to a bifunctional or higher isocyanate in UV absorption (210 nm), and (B) is an area of the peak corresponding to a compound having UV absorption (wavelength: 254 nm) in the region of a decamer or higher isocyanate.)

A compound having UV absorption (wavelength: 254 nm) in the region of a decamer or higher isocyanate is an impurity component having a high boiling point, and when such a compound is produced in small amounts, storage stability can be evaluated to improve.

The peak area ratio (%) after 300 days of storage was measured by GPC using polyisocyanate compositions after completion of the storage period as samples. For the storage method, a method was employed in which 300 g of the polyisocyanate composition obtained above was placed in a 500 mL SUS storage container, and following nitrogen substitution, stored for 300 days in a storage environment in the Kojima district of Kurashiki City, Okayama Prefecture, Japan.

In Example 18 and Comparative Example 2, the storage container was changed to a glass bottle, and the storage location was changed to outdoors.

### (GPC measurement method)

Equipment used: HLC-8120 (manufactured by Tosoh Corporation)
Columns used: TSK GEL Super H1000, TSK GEL Super H₂000, TSK GEL Super H3000 (all manufactured by Tosoh Corporation)
Sample concentration: 5 wt/vol% (50 mg of sample dissolved in 1 mL of tetrahydrofuran (THF))
Carrier: THF
Detection method: PDA detector
Discharge: 0.6 mL/min
Column temperature: 30°C
Polystyrene with a molecular weight of 1,000 to 20,000 was used to produce a calibration curve.

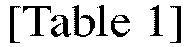

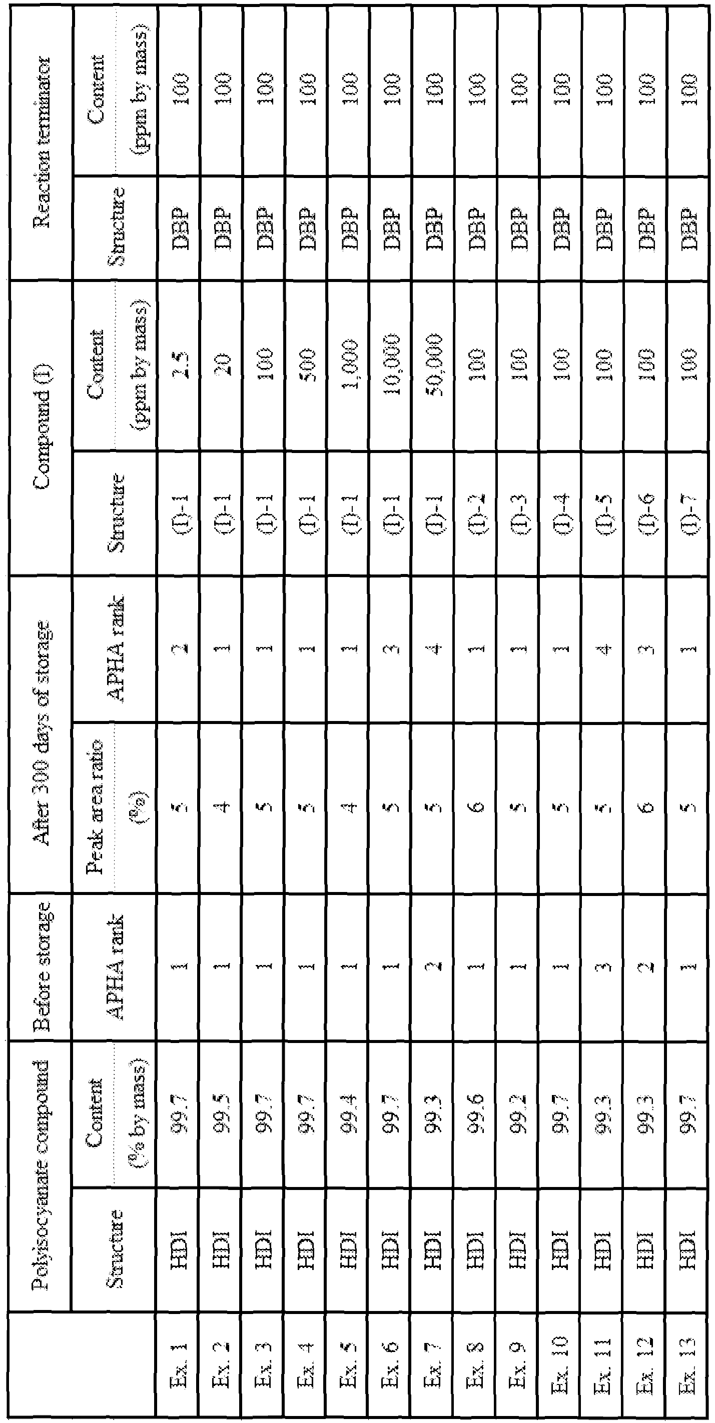

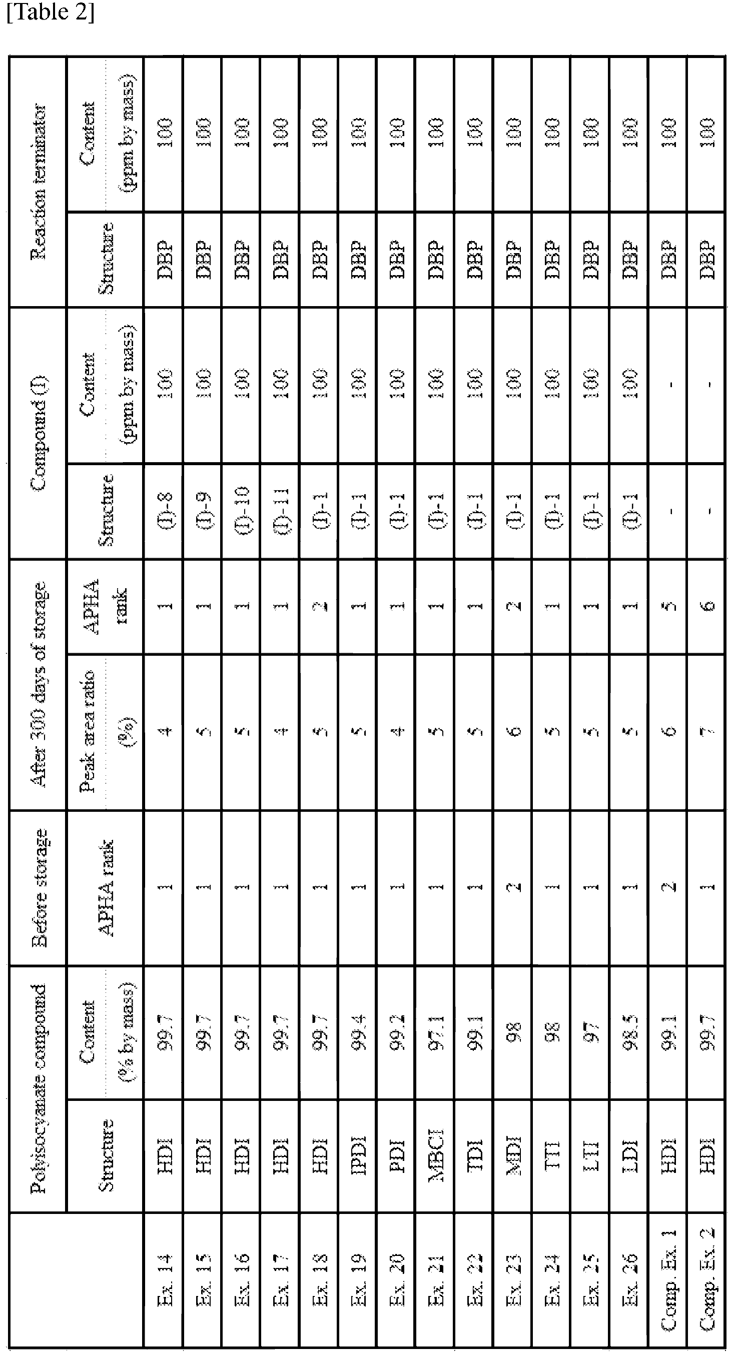

Tables 1 and 2 show that the polyisocyanate composition 1 containing a compound selected from the group consisting of the compounds (1) exhibited excellent storage stability and coloration inhibition.

The polyisocyanate compositions containing no compound (I) gelled during the 300-day storage period, making the GPC measurement impossible.

### <Production of polyisocyanate composition 2>

A polyisocyanate composition 2 containing a quinazolinedione compound and a polyisocyanate compound was produced.

More specifically, as shown in Table 3, the polyisocyanate composition 2 was prepared using any one of HDI, IPDI, PDI, MBCI, TDI, MDI, TTI, LTI, and LDI as the polyisocyanate compound.

Any one of a compound derived from N-methylaniline (NMA), a compound derived from N-ethylaniline (NEA), a compound derived from N-butylaniline (NBA), and a compound derived from N-isopropylaniline (NiPA), which was a compound obtained by reacting a secondary amine compound with a carbonic acid derivative, was used as the quinazolinedione compound.

In the compound derived from NMA, in the above general formula (II), R²¹⁰ is a methyl group, and R²²⁰, R²³⁰, R²⁴⁰, and R²⁵⁰ are hydrogen.

In the compound derived from NEA, in the above general formula (II), R²¹⁰ is an ethyl group, and R²²⁰, R²³⁰, R²⁴⁰, and R²⁵⁰ are hydrogen.

In the compound derived from NBA, in the above general formula (II), R²¹⁰ is an n-butyl group, and R²²⁰, R²³⁰, R²⁴⁰, and R²⁵⁰ are hydrogen.

In the compound derived from NiPA, in the above general formula (II), R²¹⁰ is an isopropyl group, and R²²⁰, R²³⁰, R²⁴⁰, and R²⁵⁰ are hydrogen.

APHA ranking before storage was carried out using the same method as that described in the above section <Evaluation of coloration inhibition>.

Storage stability was evaluated using the same method as that described in the above section <Evaluation of storage stability>.

### <Evaluation of APHA rank after storage>

The APHA rank after 300 days of storage was measured using a sample prepared by dissolving 1 g of the polyisocyanate composition after 300 days of storage in 2 g of benzyltoluene, using the method described in the above section <Evaluation of coloration inhibition>. Based on the values measured using a Hazen meter, ranking was carried out using the same evaluation criteria as described in the above section

### <Evaluation of coloration inhibition>.

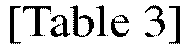

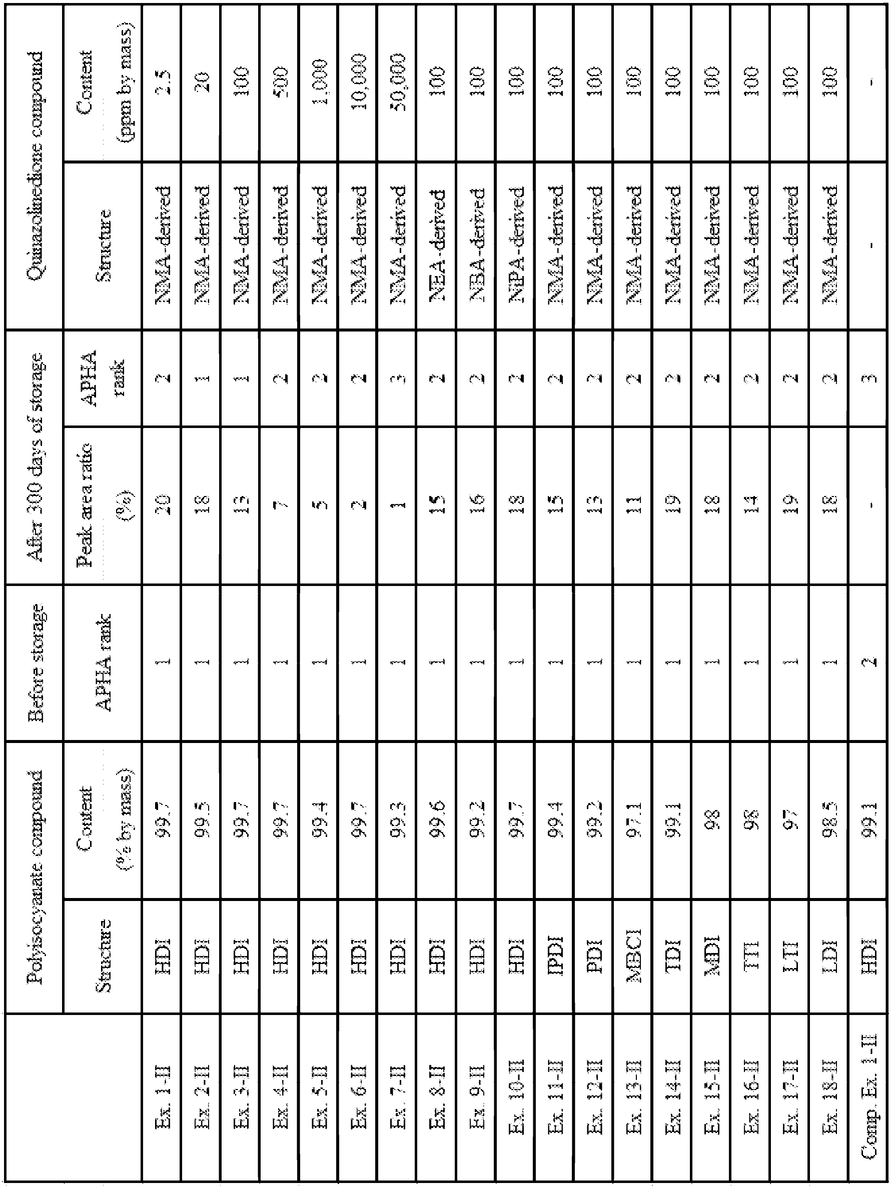

Table 3 shows that the polyisocyanate composition 2 containing a compound selected from the group consisting of the quinazolinedione compounds (II) exhibited excellent storage stability and coloration inhibition.

The polyisocyanate compositions containing no quinazolinedione compound (II) gelled during the 300-day storage period, making the GPC measurement impossible.

### <Production of polyisocyanate composition 3>

A polyisocyanate composition 3 containing a trisubstituted urea compound and a polyisocyanate compound was produced.

More specifically, as shown in Table 4, the polyisocyanate composition 3 was prepared using any one of HDI, IPDI, PDI, MBCI, TDI, MDI, TTI, LTI, and LDI as the polyisocyanate compound.

A compound obtained by reacting at least one isocyanate group of a polyisocyanate compound with a secondary amine compound was used as the trisubstituted urea compound. Any one of N-methylaniline (NMA), N-ethylaniline (NEA), N-butylaniline (NBA), N-isopropylaniline (NiPA), diethylamine (DEA), and diphenylamine (DPA) was used as the secondary amine compound.

A trisubstituted urea compound obtained using HDI is represented by the above general formula (1).

A trisubstituted urea compound obtained using IPDI is represented by the above general formula (2).

A trisubstituted urea compound obtained using PDI is represented by the above general formula (3).

A trisubstituted urea compound obtained using MBCI is represented by the above general formula (4).

Trisubstituted urea compounds obtained using TDI are represented by the above general formulas (5) and (6).

A trisubstituted urea compound obtained using MDI is represented by the above general formula (7).

A trisubstituted urea compound obtained using TTI is represented by the above general formula (8).

A trisubstituted urea compound obtained using LTI is represented by the above general formula (9).

A trisubstituted urea compound obtained using LDI is represented by the above general formula (10).

In the trisubstituted urea compound obtained by reacting a polyisocyanate compound with NMA, in the above general formula (III), one of R³²⁰ and R³³⁰ is a methyl group and the other is a phenyl group.

In the trisubstituted urea compound obtained by reacting a polyisocyanate compound with NEA, in the above general formula (III), one of R³²⁰ and R³³⁰ is an ethyl group and the other is a phenyl group.

In the trisubstituted urea compound obtained by reacting a polyisocyanate compound with NBA, in the above general formula (III), one of R³²⁰ and R³³⁰ is an n-butyl group and the other is a phenyl group.

In the trisubstituted urea compound obtained by reacting a polyisocyanate compound with NiPA, in the above general formula (III), one of R³²⁰ and R³³⁰ is an isopropyl group and the other is a phenyl group.

In the trisubstituted urea compound obtained by reacting a polyisocyanate compound with DEA, in the above general formula (III), both of R³²⁰ and R³³⁰ are ethyl groups.

In the trisubstituted urea compound obtained by reacting a polyisocyanate compound with DPA, in the above general formula (III), both of R³²⁰ and R³³⁰ are phenyl groups.

APHA ranking before storage was carried out using the same method as that described in the above section <Evaluation of coloration inhibition>.

Storage stability was evaluated using the same method as that described in the above section <Evaluation of storage stability>.

### <Evaluation of APHA rank after storage>

The APHA rank after 300 days of storage was measured using a sample prepared by dissolving 1 g of the polyisocyanate composition after 300 days of storage in 2 g of benzyltoluene, using the method described in the above section <Evaluation of coloration inhibition>. Based on the values measured using a Hazen meter, ranking was carried out using the same evaluation criteria as described in the above section

### <Evaluation of coloration inhibition>.

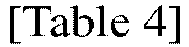

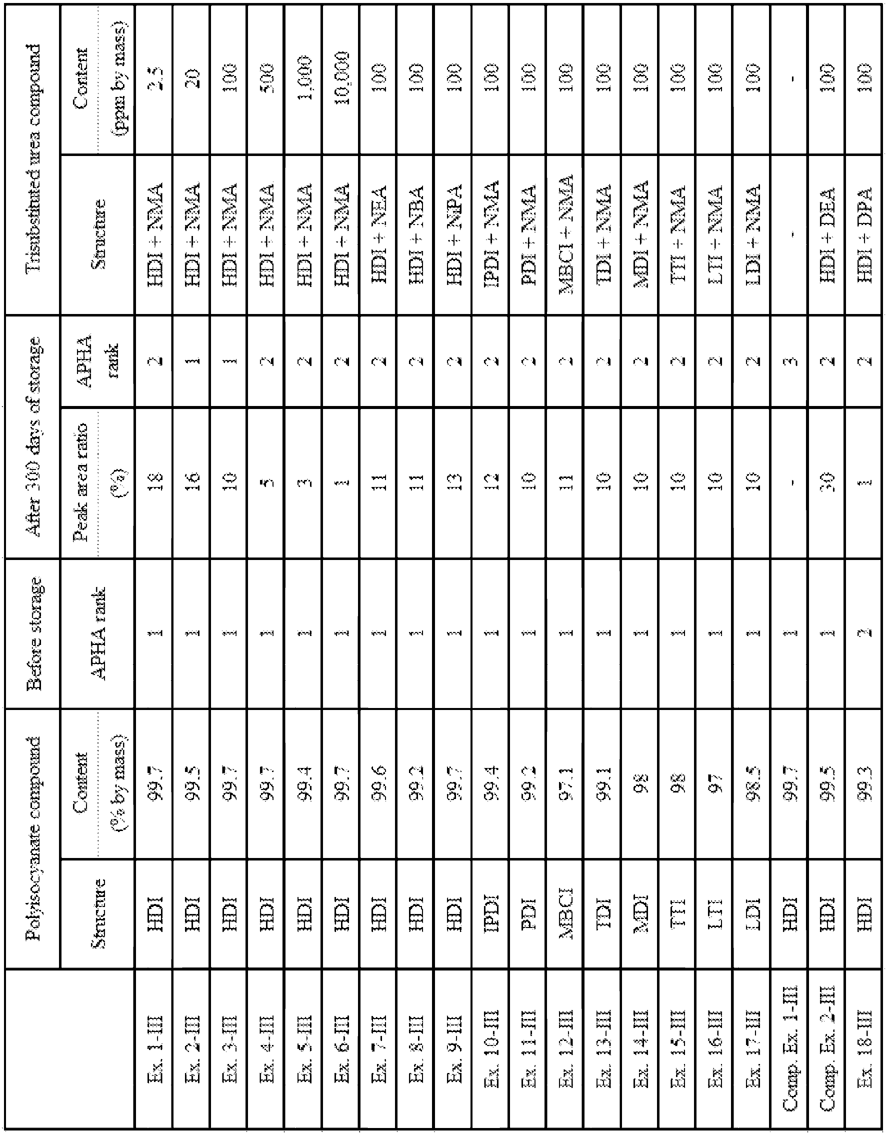

Table 4 shows that the polyisocyanate composition 3 containing a compound selected from the group consisting of the trisubstituted urea compounds (III) in which at least one of R³²⁰ and R³³⁰ has an aromatic group exhibited excellent storage stability and coloration inhibition.

The polyisocyanate compositions containing no trisubstituted urea compound (III) gelled during the 300-day storage period, making the GPC measurement impossible.

The polyisocyanate compositions containing a trisubstituted urea compound in which neither R³²⁰ nor R³³⁰ was an aromatic group exhibited poor storage stability.

### <Production of polyisocyanate composition 4>

A polyisocyanate composition 4 containing two or more of the compound (I), the quinazolinedione compound (II), and the trisubstituted urea compound (III) was produced.

As shown in Table 5, the polyisocyanate composition 4 was prepared using any one of HDI, IPDI, PDI, MBCI, TDI, MDI, TTI, LTI, and LDI as the polyisocyanate compound. Here, the compound (I), the quinazolinedione compound (II), and the trisubstituted urea compound (III) were prepared in the same manner as in the production of polyisocyanate compositions 1 to 3.

APHA ranking before storage was carried out using the same method as that described in the above section <Evaluation of coloration inhibition>.

Storage stability was evaluated using the same method as that described in the above section <Evaluation of storage stability>.

### <Evaluation of APHA rank after storage>

The APHA rank after 300 days of storage was measured using a sample prepared by dissolving 1 g of the polyisocyanate composition after 300 days of storage in 2 g of benzyltoluene, using the method described in the above section <Evaluation of coloration inhibition>. Based on the values measured using a Hazen meter, ranking was carried out using the same evaluation criteria as described in the above section

### <Evaluation of coloration inhibition>.

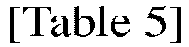

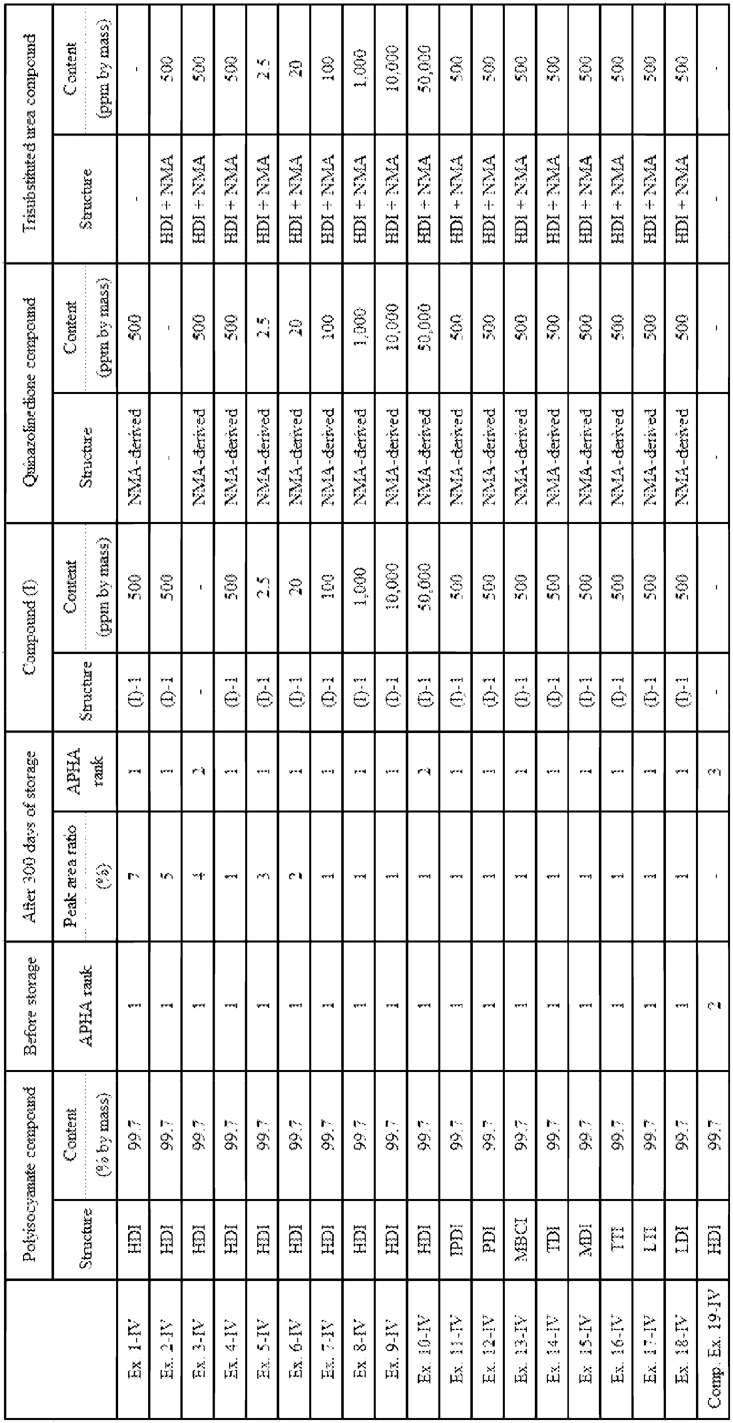

Table 5 shows that the polyisocyanate composition 4 containing two or more of the compound (I), the quinazolinedione compound (II), and the trisubstituted urea compound (III) exhibited excellent storage stability and coloration inhibition. In particular, in Examples 4-IV to 10-IV containing the compound (I), the quinazolinedione compound (II), and the trisubstituted urea compound (III), peak area ratios (%) were 3 or less and APHA ranks were 2 or less, exhibiting particularly excellent storage stability and coloration inhibition.

The polyisocyanate composition (Comparative Example 19-IV) containing none of the compound (I), the quinazolinedione compound (II), and the trisubstituted urea compound (III) gelled during the 300-day storage period, making the GPC measurement impossible.

### <Isocyanate compound production method 1>

An isocyanate compound was produced in the presence of the compound (I).

### [Production example of blocked isocyanate compound]

20 parts by mass of hexamethylene diisocyanate, 80 parts by mass of phenol, and 0.1 parts by mass of water were mixed and reacted at 150°C for 20 hours to synthesize diphenylhexane-1,6-diyldicarbamate. Subsequently, phenol present in an excessive amount was remove by distillation under reduced pressure at 100°C and 1 Pa. A blocked isocyanate composition BL-1 containing 5 mol% of ureylene groups and 95 mol% of blocked isocyanate groups was obtained as a result of 1H NMR analysis.

Table 6 shows the abbreviations for the obtained blocked isocyanate compounds, as well as the contents of ureylene (-NHCONH-) groups and blocked isocyanate (BL-NCO) groups.

### [Examples 1A to 15A]

The blocked isocyanate compound from the aforementioned production example, the compound (I), and a solvent were added as shown in Table 7 into a 300 mL glass container equipped with a pressure reducing device at the end of a condenser tube 1 (having a structure designed so that a condensate in the condenser tube 1 enters the 300 mL glass container) and a condenser tube 2 (a condensate in the condenser tube 2 does not enter the 300 mL glass container but is recovered as a TOP liquid) that are filled with a filler. Subsequently, the temperature inside the 300 mL glass container (reaction temperature), the degree of pressure reduction, the temperature of the condenser tube 1, and the temperature of the condenser tube 2 were set as shown in the reaction conditions in Table 2. The absolute pressure inside the reaction vessel was adjusted to 380 mmHg using the pressure reducing device, and the reaction was then allowed to proceed for 3 hours.

The yield of isocyanate groups (NCO group mol%) and the residual ratio of ureylene groups (ureylene group mol%) contained in the obtained reaction liquid were determined.

### [Comparative Examples 1A to 11A]

A blocked isocyanate compound was subjected to heat treatment in the same manner as in Examples 1A to 15A, with the exception that, as shown in Table 8, the blocked isocyanate compound and a solvent were added into a glass container without adding the compound (1) to set the reaction conditions.

It should be noted that in the following tables, the abbreviations refer to the following compounds.

### (Isocyanate compounds)

HDI: 1,6-hexamethylene diisocyanate
IPDI: isophorone diisocyanate (mixture of isomers)
TDI: diisocyanatotoluene (mixture of isomers)
MDI: diphenylmethane diisocyanate (mixture of isomers)
HMDI: dicyclohexylmethane 4,4'-diisocyanate

### (Blocking agents)

PhOH: phenol
o-cresol: o-cresol
m-cresol: m-cresol
p-cresol: p-cresol
n-BuOH: normal butanol
DBA: dibutylamine
NMA: N-methylaniline

### (Solvent)

Naphthene: naphthenic solvent (EXXOLTMD80)

In the following Table 6, the structure of the compound (I) is as follows.

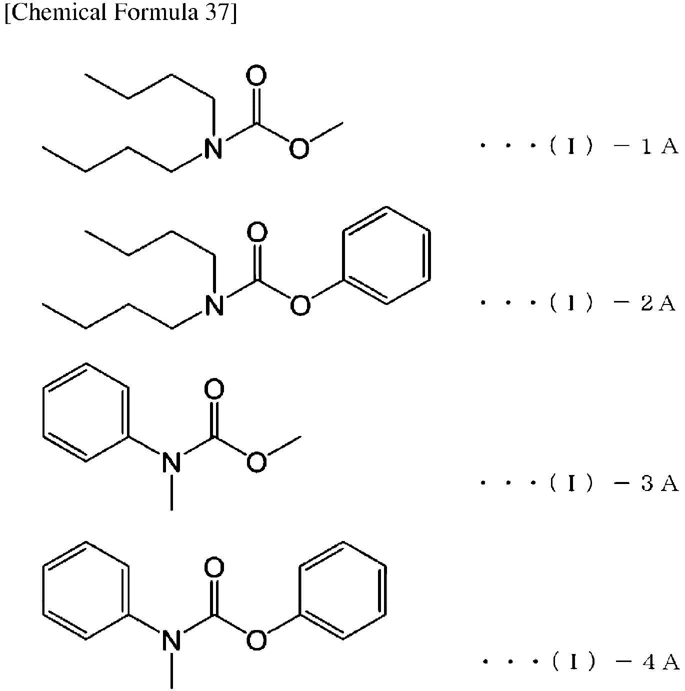

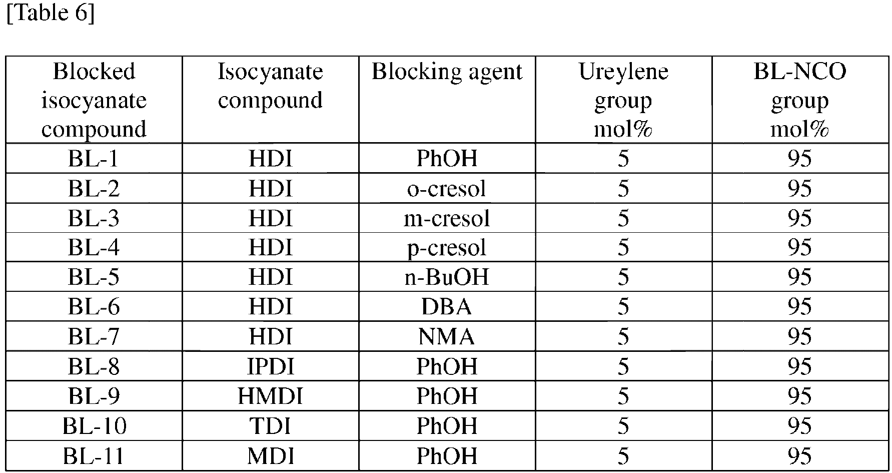

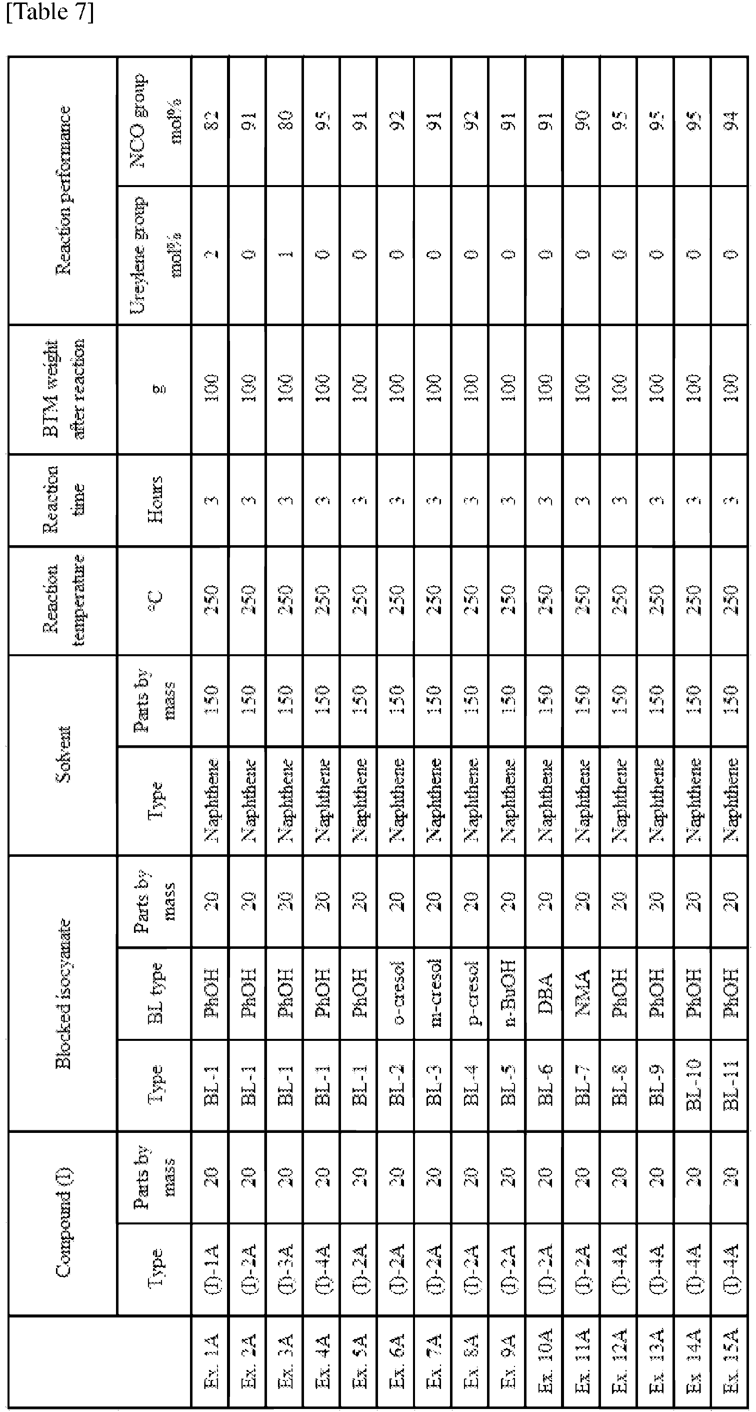

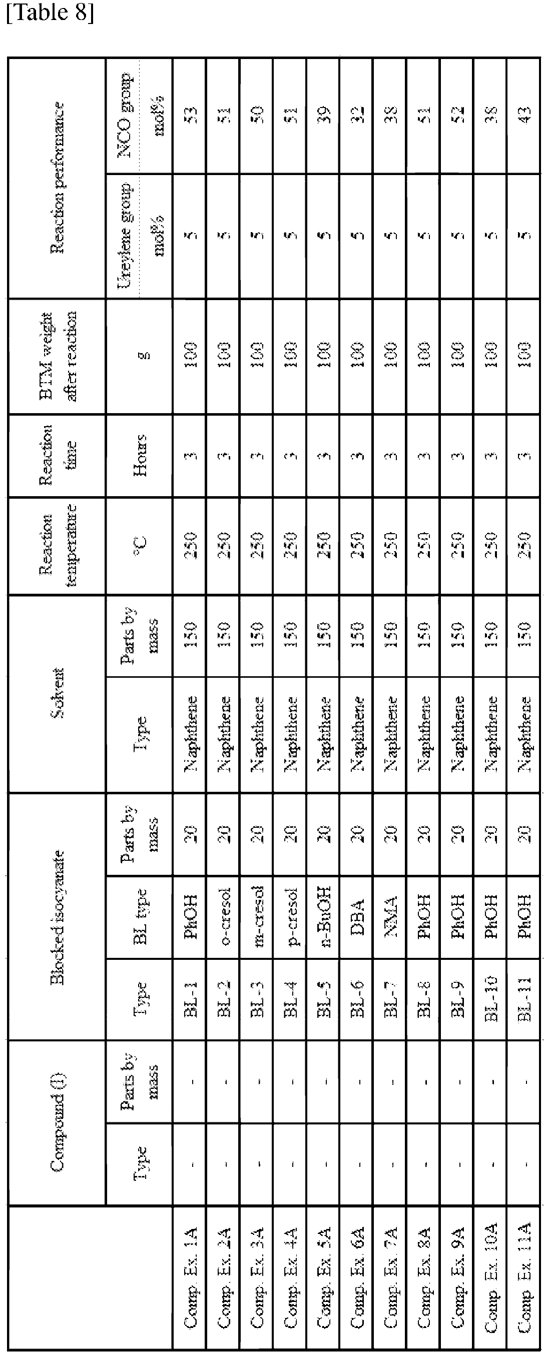

Table 7 shows that when a blocked isocyanate compound was thermally decomposed in the presence of the compound (I), the residual ratio of ureylene groups (ureylene group mol%) decreased and the yield of isocyanate groups (NCO group mol%) was improved.

On the other hand, Table 8 shows that when a blocked isocyanate compound was thermally decomposed without the presence of the compound (I), the residual ratio of ureylene groups remained unchanged and the yield of isocyanate groups decreased.

### <Isocyanate compound production method 2>

An isocyanate compound was produced in the presence of the compound (II).

### [Production example of blocked isocyanate compound]

One part by mass of an isocyanate compound and 9 parts by mass of a blocking agent were added into a SUS pressure resistant vessel and reacted at 150°C for 20 hours. The free blocking agent contained in the obtained reaction liquid was removed by distillation under reduced pressure to obtain a blocked isocyanate composition.

Table 9 shows the abbreviations for the obtained blocked isocyanate compounds and the content of blocked isocyanate (BL-NCO) groups.

### [Examples 1B to 17B]

The blocked isocyanate compound from the aforementioned production example, a catalyst corresponding to the compound (II), and a solvent were added as shown in Table 10 into a 300 mL glass container equipped with a pressure reducing device at the end of a condenser tube 1 (having a structure designed so that a condensate in the condenser tube 1 enters the 300 mL glass container) and a condenser tube 2 (a condensate in the condenser tube 2 does not enter the 300 mL glass container but is recovered as a TOP liquid) that are filled with a filler. Subsequently, the temperature inside the 300 mL glass container (reaction temperature), the degree of pressure reduction, the temperature of the condenser tube 1, and the temperature of the condenser tube 2 were set as shown in the reaction conditions in Table 2, and the reaction was then allowed to proceed for three hours.

The yield of isocyanate groups (NCO group mol%) and the residual ratio of blocked isocyanate groups (BL-NCO group mol%) contained in the obtained reaction liquid were determined. Further, the sum of the yield of isocyanate groups and the residual ratio of blocked isocyanate groups was determined as a mass balance (MB mol%).

### [Comparative Examples 1B to 13B]

A blocked isocyanate compound was subjected to heat treatment in the same manner as in Examples 1B to 17B, with the exception that, as shown in Table 11, the blocked isocyanate compound and a solvent were added into a glass container without adding a catalyst to set the reaction conditions.

### [Comparative Example 14B]

A blocked isocyanate compound was subjected to heat treatment in the same manner as in Examples 1B to 17B, with the exception that, as shown in Table 11, the blocked isocyanate compound, a catalyst, and a solvent were added into a glass container to set the reaction conditions.

It should be noted that in the following tables, the abbreviations refer to the following compounds.

### (Isocyanate compounds)

HDI: 1,6-hexamethylene diisocyanate
IPDI: isophorone diisocyanate (mixture of isomers)
TDI: diisocyanatotoluene (mixture of isomers)
MDI: diphenylmethane diisocyanate (mixture of isomers)
HMDI: dicyclohexylmethane 4,4'-diisocyanate

### (Blocking agents)

PhOH: phenol
o-cresol: o-cresol
m-cresol: m-cresol
p-cresol: p-cresol
n-BuOH: normal butanol
DBA: dibutylamine
NMA: N-methylaniline

### (Catalyst: compound (II))

MQD: 1-methyl-2,4(1H, 3H)-quinazolinedione
BQD: 1-butyl-2,4(1H, 3H)-quinazolinedione
PQD: 1-phenyl-2,4(1H, 3H)-quinazolinedione

### (Solvent)

DBT: dibenzyltoluene

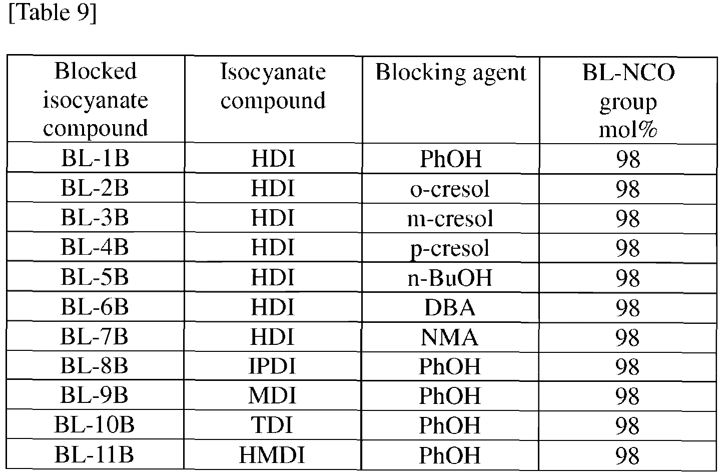

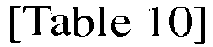

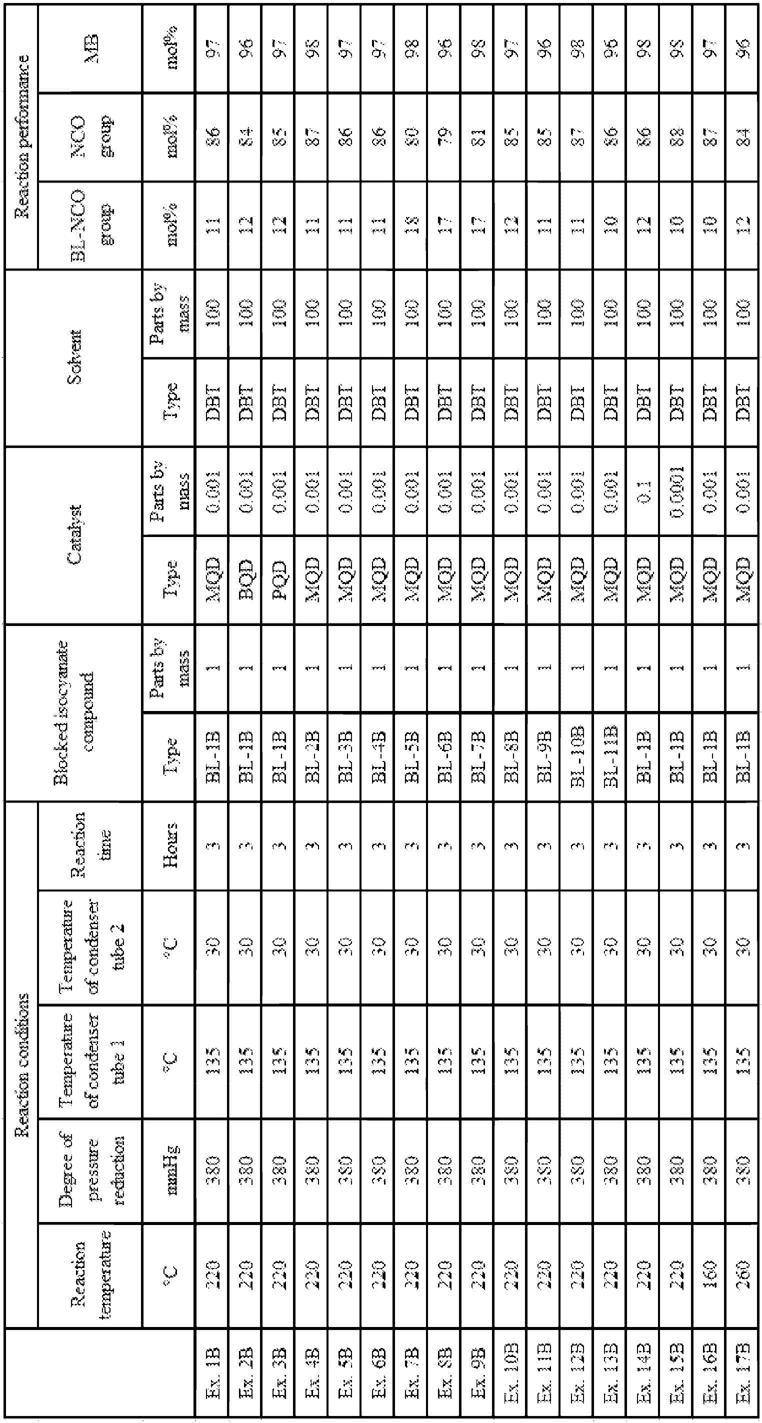

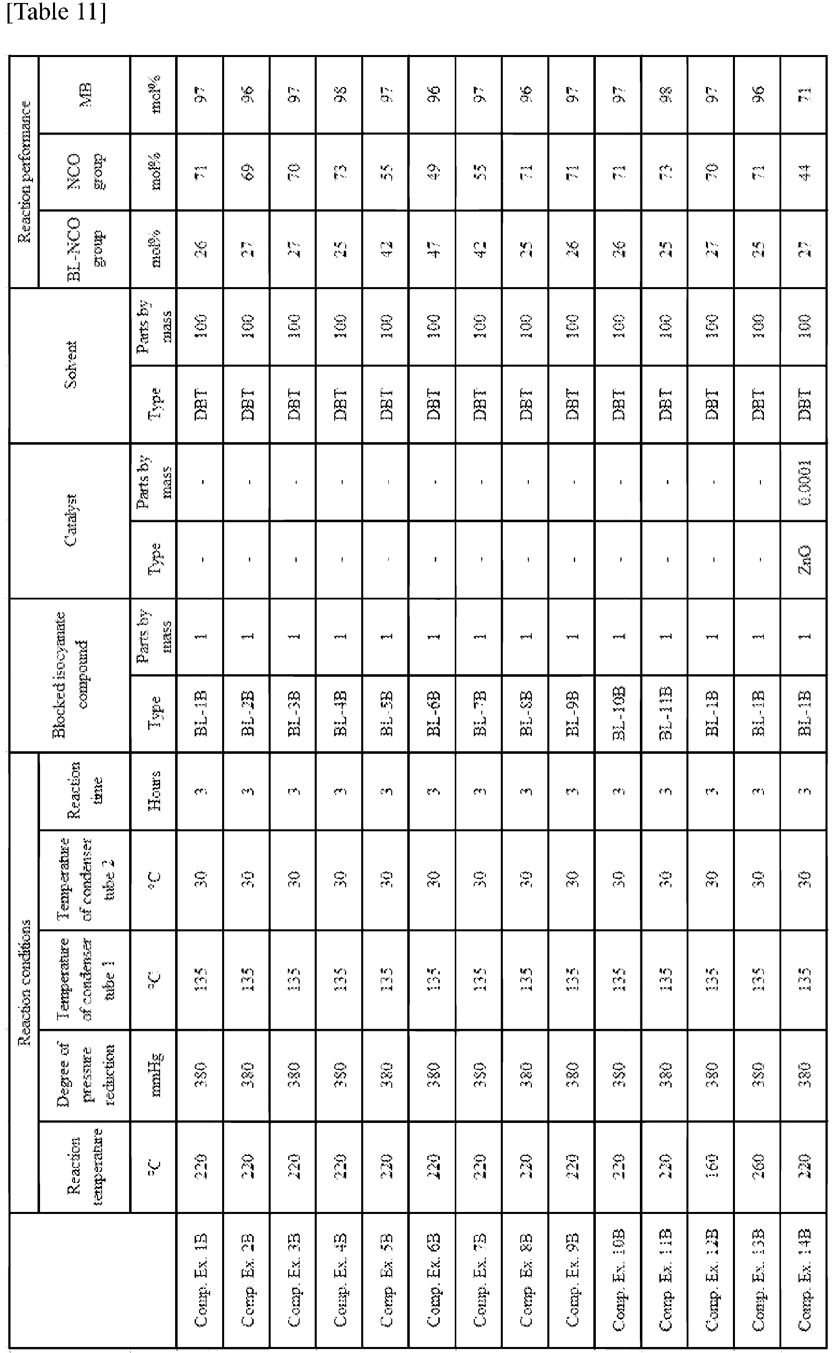

Table 10 shows that when a blocked isocyanate compound was thermally decomposed in the presence of a compound having the quinazolinedione structure (II), the residual ratio of blocked isocyanate groups (BL-NCO groups mol%) decreased and the yield of isocyanate groups (NCO group mol%) was improved.

### INDUSTRIAL APPLICABILITY

According to the polyisocyanate compositions 1 to 4 of the present embodiment, it is possible to provide a polyisocyanate composition with excellent storage stability and coloration inhibition.

According to the Isocyanate Compound Production Methods 1 to 2 of the present embodiment, it is possible to provide a method for producing an isocyanate compound that can improve the thermal decomposition rate of a blocked isocyanate compound without increasing the amount of by-products produced.

## Claims

1. A polyisocyanate composition comprising at least one or more compounds represented by any one of the following general formulas (I), (II), and (III), and a polyisocyanate compound, 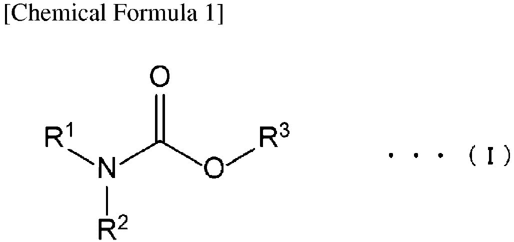 (wherein R¹ and R² each independently represent a monovalent organic group; R¹ and R² may each independently form a ring structure via a carbon-carbon bond, a carbon-oxygen-carbon bond, or a carbon-nitrogen-carbon bond; and R³ represents an aliphatic hydrocarbon group or a hydrocarbon group having an aromatic group), 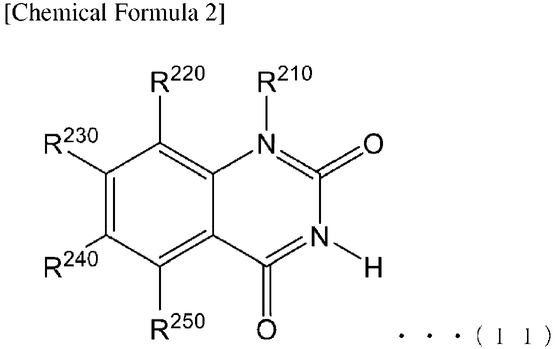 (wherein R²¹⁰ represents a monovalent organic group, and R²²⁰, R²³⁰, R²⁴⁰, and R²⁵⁰ each independently represent a monovalent organic group or hydrogen; and R²¹⁰ and R²²⁰ may be bonded to each other to form a ring structure via a carbon-carbon bond, a carbon-oxygen-carbon bond, or a carbon-nitrogen-carbon bond), 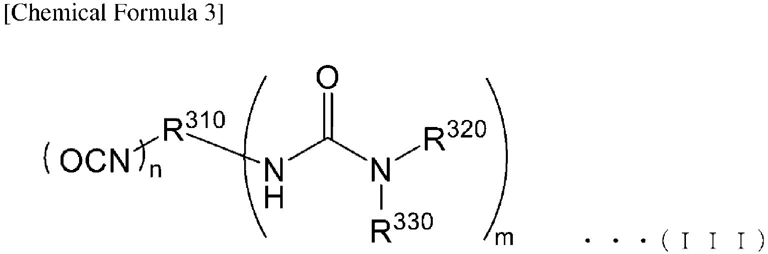 (wherein R³¹⁰ represents an (n + m )-valent organic group, n is an integer from 0 to 12, m is an integer from 1 to 12, n + m is an integer of 13 or less, and R³²⁰ and R³³⁰ each independently represent a monovalent organic group; R³²⁰ and R³³⁰ may be bonded to each other to form a ring structure via a carbon-carbon bond, a carbon-oxygen-carbon bond, or a carbon-nitrogen-carbon bond; and at least one of R³²⁰ and R³³⁰ has an aromatic group.)

2. The polyisocyanate composition according to Claim 1, comprising 1.0 ppm by mass or more and 1.0 × 10⁴ ppm by mass or less of at least one or more compounds represented by any one of said general formulas (I), (II), and (III), based on a total mass of said polyisocyanate compound.

3. A method for producing an isocyanate compound, the method comprising a reaction step of decomposing a blocked isocyanate compound into a blocking agent and an isocyanate compound by heat treatment in the presence of a compound having a structure represented by either one or both of the following general formulas (I) and (II), thereby obtaining the isocyanate compound: 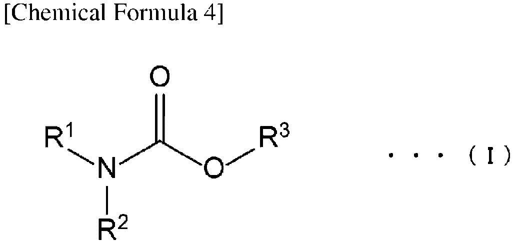 (wherein R¹ and R² each independently represent a monovalent organic group; R¹ and R² may each independently form a ring structure via a carbon-carbon bond, a carbon-oxygen-carbon bond, or a carbon-nitrogen-carbon bond; and R³ represents an aliphatic hydrocarbon group or a hydrocarbon group having an aromatic group), 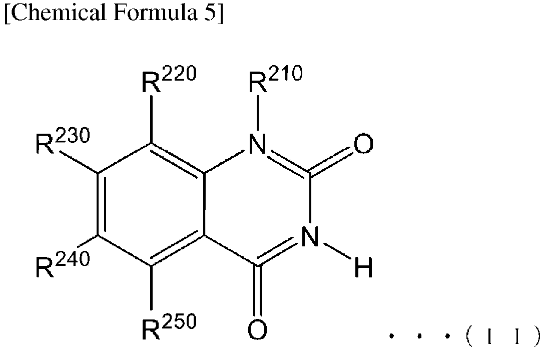 (wherein R²¹⁰ represents a monovalent organic group, and R²²⁰, R²³⁰, R²⁴⁰, and R²⁵⁰ each independently represent a monovalent organic group or hydrogen; and R²¹⁰ and R²²⁰ may be bonded to each other to form a ring structure via a carbon-carbon bond, a carbon-oxygen-carbon bond, or a carbon-nitrogen-carbon bond.)

4. The method for producing an isocyanate compound according to Claim 3,
wherein said blocking agent comprises one or more compounds selected from the group consisting of a hydroxy compound, an amine compound, and ammonia.

5. The method for producing an isocyanate compound according to Claim 3 or 4, wherein said isocyanate compound is an isocyanate compound represented by the following general formula (IV): 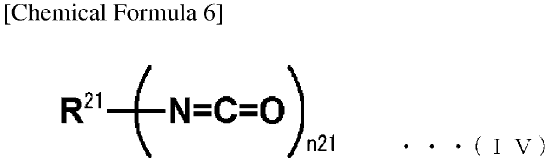 (wherein R²¹ is an n21-valent organic group; and n21 is an integer from 1 to 12.)

6. The method for producing an isocyanate compound according to Claim 3 or 4, wherein said blocking agent is an aromatic hydroxy compound represented by the following general formula (V): 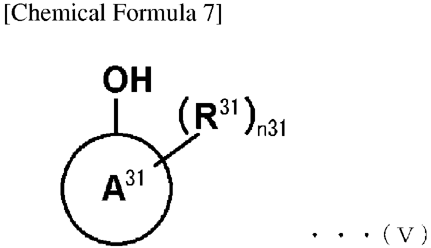 (wherein a ring A³¹ represents an aromatic hydrocarbon ring having 6 to 20 carbon atoms; R³¹ represents a hydrogen atom, a halogen atom, a carboxy group, an alkyl group having 1 to 20 carbon atoms, an alkoxy group having 1 to 20 carbon atoms, an alkyloxycarbonyl group having 1 to 20 carbon atoms, an alkylcarbonyloxy group having 1 to 20 carbon atoms, an aryl group having 6 to 20 carbon atoms, an aryloxy group having 6 to 20 carbon atoms, an aralkyl group having 7 to 20 carbon atoms, or an aralkyloxy group having 7 to 20 carbon atoms; R³¹ may be bonded with the ring A³¹ to form a ring structure; and n31 is an integer from 1 to 10.)

7. The method for producing an isocyanate compound according to Claim 3 or 4, wherein said blocking agent is an aliphatic hydroxy compound represented by the following general formula (VI): 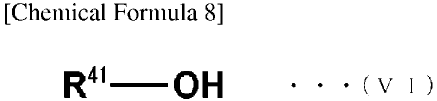 (wherein R⁴¹ represents a substituted or unsubstituted aliphatic hydrocarbon group having 1 to 24 carbon atoms, which may have an ether group, a carbonyl group, or an ester group.)

8. The method for producing an isocyanate compound according to Claim 3 or 4, wherein said blocking agent is a secondary amine compound represented by the following general formula (VII): 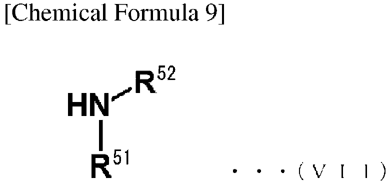 (wherein R⁵¹ and R⁵² each independently represent a monovalent organic group; R⁵¹ and R⁵² may be bonded to each other to form a ring structure via a carbon-carbon bond, a carbon-oxygen-carbon bond, or a carbon-nitrogen-carbon bond.)

9. The method for producing an isocyanate compound according to Claim 3 or 4, wherein in said reaction step, an amount of a compound having a structure represented by either one or both of said general formulas (I) and (II) present is 1 ppm by mass or more with respect to said blocked isocyanate compound.
